(19) Europäisches Patentamt
European Patent Office
Office européen des brevets

(11) **EP 3 015 114 B1**

(12) **EUROPEAN PATENT SPECIFICATION**

(45) Date of publication and mention
of the grant of the patent:
**12.07.2023 Bulletin 2023/28**

(21) Application number: **14895356.5**

(22) Date of filing: **26.11.2014**

(51) International Patent Classification (IPC):
**A61K 9/107** (2006.01)     **A61K 39/39** (2006.01)
**A61K 39/145** (2006.01)    **A61K 47/44** (2017.01)
**A61K 47/14** (2017.01)      **A61K 47/22** (2006.01)
**A61K 47/06** (2006.01)      **A61P 37/02** (2006.01)

(52) Cooperative Patent Classification (CPC):
**A61K 39/39; A61K 9/1075; A61K 39/12;**
**A61K 47/02; A61K 47/34; A61K 47/36;**
A61K 9/146; A61K 9/5031; A61K 47/06;
A61K 47/22; A61K 47/44; A61K 2039/5252;
A61K 2039/543; A61K 2039/55505;
A61K 2039/55555;                              (Cont.)

(86) International application number:
**PCT/CN2014/092290**

(87) International publication number:
**WO 2015/192603 (23.12.2015 Gazette 2015/51)**

(54) **OIL-IN-WATER EMULSION CONTAINING NO SURFACTANT AND USE THEREOF**

TENSIDFREIE ÖL-IN-WASSER-EMULSION UND VERWENDUNG DAVON

ÉMULSION HUILE DANS EAU NE CONTENANT PAS DE TENSIOACTIF, ET SON UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.06.2014 CN 201410272743**

(43) Date of publication of application:
**04.05.2016 Bulletin 2016/18**

(73) Proprietor: **Institute Of Process Engineering
Chinese Acadamy Of Sciences
Haidian District, Beijing 100190 (CN)**

(72) Inventors:
• **MA, Guanghui**
**Beijing 100190 (CN)**
• **WU, Jie**
**Beijing 100190 (CN)**
• **YANG, Liuqing**
**Beijing 100190 (CN)**
• **XIA, Yufei**
**Beijing 100190 (CN)**
• **QI, Feng**
**Beijing 100190 (CN)**
• **FAN, Qingze**
**Beijing 100190 (CN)**

(74) Representative: **Rapisardi, Mariacristina
Ufficio Brevetti Rapisardi S.r.l.
Via Serbelloni, 12
20122 Milano (IT)**

(56) References cited:
CN-A- 1 171 052          CN-A- 1 249 926
CN-A- 1 440 741          CN-A- 101 677 913
CN-A- 104 013 955       US-A- 5 667 784
US-A1- 2011 052 633

• FRELICHOWSKA J ET AL: "Topical delivery of
lipophilic drugs from o/w Pickering emulsions",
INTERNATIONAL JOURNAL OF
PHARMACEUTICS, ELSEVIER BV, NL, vol. 371,
no. 1-2, 17 April 2009 (2009-04-17), pages 56-63,
XP026071362, ISSN: 0378-5173, DOI:
10.1016/J.IJPHARM.2008.12.017 [retrieved on
2009-03-25]

- CHEVALIER Y ET AL: "Emulsions stabilized with solid nanoparticles: Pickering emulsions", COLLOIDS AND SURFACES A: PHYSICOCHEMICAL AND ENGINEERING ASPECTS 20131220 ELSEVIER NLD, vol. 439, 20 December 2013 (2013-12-20), pages 23-34, XP028764672, ISSN: 0927-7757
- WAHLGREN MARIE ET AL: "The use of micro- and nanoparticles in the stabilisation of pickering-type emulsions for topical delivery.", CURRENT PHARMACEUTICAL BIOTECHNOLOGY 2013, vol. 14, no. 15, 2013, pages 1222-1234, XP009192100, ISSN: 1873-4316
- WHITBY CATHERINE P ET AL: "Poly(lactic-co-glycolic acid) as a particulate emulsifier.", JOURNAL OF COLLOID AND INTERFACE SCIENCE 1 JUN 2012, vol. 375, no. 1, 1 June 2012 (2012-06-01), pages 142-147, XP028478208, ISSN: 0021-9797
- NAN F ET AL: "Uniform chitosan-coated alginate particles as emulsifiers for preparation of stable Pickering emulsions with stimulus dependence", COLLOIDS AND SURFACES A: PHYSICOCHEMICAL AND ENGINEERING ASPECTS 20140820 ELSEVIER NLD, vol. 456, no. 1, 20 August 2014 (2014-08-20), pages 246-252, XP028860859, ISSN: 0927-7757
- WANG XIAOLI ET AL: "Preparation of core-shell CaCO3 capsules via Pickering emulsion templates.", JOURNAL OF COLLOID AND INTERFACE SCIENCE 15 APR 2012, vol. 372, no. 1, 15 April 2012 (2012-04-15), pages 24-31, XP028461909, ISSN: 0021-9797
- MARKU DIANA ET AL: "Characterization of starch Pickering emulsions for potential applications in topical formulations.", INTERNATIONAL JOURNAL OF PHARMACEUTICS 30 MAY 2012, vol. 428, no. 1-2, 30 May 2012 (2012-05-30), pages 1-7, XP055311688, ISSN: 0378-5173
- LAREDJ-BOUREZG F ET AL: "Emulsions stabilized with organic solid particles", COLLOIDS AND SURFACES A: PHYSICOCHEMICAL AND ENGINEERING ASPECTS 20121105 ELSEVIER NLD, vol. 413, 5 November 2012 (2012-11-05), pages 252-259, XP028943889, ISSN: 0927-7757
- FOX CHRISTOPHER B ET AL: "An update on safety and immunogenicity of vaccines containing emulsion-based adjuvants.", EXPERT REVIEW OF VACCINES JUL 2013, vol. 12, no. 7, July 2013 (2013-07), pages 747-758, XP009192069, ISSN: 1744-8395

Remarks:

The file contains technical information submitted after the application was filed and not included in this specification

(52) Cooperative Patent Classification (CPC): (Cont.)
A61K 2039/55566; A61K 2039/55583;
C12N 2730/10134; C12N 2760/16134;
C12N 2770/32134

## Description

### Technical Field

The present invention relates to a biological product, especially an oil-in-water emulsion applicable in human or other animal bodies. It is particularly important that the oil-in-water emulsion of the present invention comprises no surfactant, and uses solid particles as the emulsion stabilizer. It can be used as a vaccine adjuvant, or as a drug delivery carrier or a sustained/controlled-release carrier, and belongs to the technical field of biological medicine.

### Background Art

With the rapid development of modern biotechnology and the increasing social emphasis on untoward effect resulted from attenuated vaccines, split vaccine, recombinant subunit vaccine, antiidiotype antibody vaccine, nucleic acid vaccine and synthetic peptide vaccine have been developed. These vaccines have a high antigen purity, a low relative molecular weight and a low untoward effect. However, these antigens generally have a weaker immunogenicity and immunoprotection. In order to improve the effectiveness of antigens, adjuvants must be added into antigens to increase their capability of inducing immunoreaction.

The simultaneous or synergistic application of adjuvants and antigens (being successively inoculated to the same or adjacent part in a shorter interval time (generally 1 h or less)) can enhance the immune response ability of organisms to antigens, or change the immunoreaction type. The functions thereof primarily include recruiting immune cells and immune molecules at the application sites and enhancing the immune response; increasing the vaccine antigen delivery; enhancing the immune contact; increasing the immunogenicity and immunological memory of weak antigens, e.g. highly-purified antigens or recombinant antigens; reducing the dose and number of times of inoculation of antigens; promoting the immune effect of vaccines in groups having a weak immune response ability; speeding up the immune response and prolonging the time of duration; changing the antigen configuration; increasing the diversity of antibody types to realize the cross-protection from pathogens which are easy to vary (e.g. influenza virus); changing the types of humoral antibodies, the affinities of IgG subclasses and antibodies and stimulating the cellular immunity and mucosal immune effect.

Aluminium adjuvant is the main adjuvant which is currently and generally approved to be used for human vaccines. Since Glenny first used aluminium salts in 1926 to adsorb diphtheria toxoid, two aluminium salt adjuvants, aluminium phosphate and aluminium hydroxide, are widely used in various vaccines. However, aluminium adjuvants are mainly used for humoral immune response, and cannot induce the cellular immunity and mucosal immune effect. Especially, it has been found in recent years that repeated inoculations of vaccines containing aluminium adjuvants may result in immunosuppression and cumulative poisoning. Moreover, aluminium adjuvants have a poor immunological enhancement effect on some vaccine antigens, and the injection sites even have serious local reactions, including erythema, subcutaneous nodule, contact hypersensitivity and granuloma inflammation. Therefore looking for new vaccine adjuvants becomes a great practical problem of vaccinology.

Recently, oil emulsion adjuvants attract more attention. Many enterprises or scientific research institutes have started to study oil emulsion adjuvants, and part of the market-available and clinical oil emulsion adjuvants are listed in Table 1 below.

Table 1 Part of the market-available and clinical oil emulsion adjuvants

| Name | Research institutes | Type | Ingredients | Average particle size of emulsion droplet (nm) | Preparation method | Immunization routes | Status |
|---|---|---|---|---|---|---|---|
| MF59 | Novartis | Oil-in-water (o/w) | Squalene, polysorbate 80, sorbitan trioleate, citrate buffer | 165 | Microfluidization | Intramuscular injection | Marketing authorization |
| AS03 | GSK | Oil-in-water (o/w) | Squalene, $\alpha$-tocopherol, polysorbate 80, phosphate-buffered saline | 150 | Microfluidization | Intramuscular injection | Marketing authorization |
| AS02 | GSK | Oil-in-water (o/w) | AS03 plus MPL and QS21 | 150 | Microfluidization | Intramuscular injection | Phase III clinical |
| AF03 | Sanofi Pasteur | Oil-in-water (o/w) | Squalene, polyoxyethylene cetyl-stearylether, sorbitan oleate, mannitol, phosphate-buffered saline | 100 | phase reversion emulsification | Intramuscular injection | Phase III clinical |
| SE, MPL-SE, GLA-SE | IDRI/ Immune Design | Oil-in-water (o/w) | Squalene, phosphatidylcholine, poloxamer 188, glycerol, ammonium phosphate buffer, MPL or GLA may be incorporated | 120 | Microfluidization | Intramuscular injection | Phase I/II clinical |
| W805EC | NanoBio | Oil-in-water (o/w) | Soybean oil, polysorbate 80, cethylpyridinium chloride, ethanol | ~400 | High-speed emulsificatio | Nasal spraying | Phase I clinical |
| CoVaccine HTTM | BTG | Oil-in-water (o/w) | Squalane, polysorbate 80, phosphate-buffered saline, sucrose fatty acid sulfate esters | 135 | Microfluidization | Intramuscular injection | Phase II clinical |
| Montanide ® ISA 720 | Seppic | Water-in-oil (w/o) | Squalene, mannide monooleate | ~1000-15 00 | Double push syringe emulsification | Intramuscular or subcutaneous injection | Phase I clinical |
| Montanide ® ISA51 | Seppic | Water-in-oil (w/o) | Mineral oil, mannide monooleate | ~1000-15 00 | Double push syringe emulsification | Intramuscular or subcutaneous injection | Marketing authorization |
| NH2 | Kurume University (Japan) | Water-in-oil (w/o) | Mineral oil, sorbitan monooleate | ~1000 | Double push syringe emulsification | subcutaneous administration | Phase I clinical |

These oil emulsion adjuvants generally consist of oil and aqueous phases, and comprises at least one surfactant. The common surfactants comprise polyoxyethylene sorbitan ester surfactant (generally called Tween), sorbitan ester (generally called Span), octoxynol-9 (triton X-100 or T-octyl phenoxy polyethoxy ethanol) and lecithin, and especially Tween 80 (Polyoxyethylene sorbitan monooleate), Span 85 (Sorbitan trioleate) and triton X-100 are more applied. Surfactants are used in preparations mainly for stabilizing the emulsion and avoiding demulsification. In order to achieve such purpose, at least one surfactant is required. Moreover, the content of surfactant in the emulsion shall go beyond the content required for emulsification to maintain the emulsion stabilization, so that there are free surfactants in aqueous phase, oil phase or in both. Although surfactants used in oil emulsion adjuvants are generally biodegradable (metabolizable) and biocompatible, the application of surfactants may bring some other adverse effects. For example, oil emulsion adjuvants often have aliphatic ingredients. For example, MF59 adjuvant comprises squalene; MPL™ comprises more monophosphoryl lipid A in a deacylation form wherein multiple fatty acid chains are connected to the main chain of diglucosamine. Studies have found that aliphatic adjuvants in vaccine compositions may be incompatible with antigens containing surfactant ingredients (CN 101267835 A). Meanwhile, although the surfactants Span 85 and Tween 80 used in MF59 and AS03 are used in foods, cosmetics or for in vivo injection, they per se are not immune adjuvants and cannot function as stimulating the immune cells (CN 102293743 B), and instead, they will increase the metabolic burden of organisms. In addition, if the membrane glycoproteins in antigens are in contact with free surfactants for a long period of time, the degeneration thereof may occur (CN 101365485 A). The addition amount of surfactants shall be strictly controlled. If surfactants are added in a larger amount, hymolysis may occur.

Unlike the traditional emulsions using surfactants as stabilizers, Pickering emulsion is an emulsion system in which traditional surfactants are replaced by solid particles. The mechanism of stabilizing the emulsion mainly involves absorbing solid particles at the oil-water interface to form a single-layered or multi-layered structure of solid particles to stabilize the emulsion. By comparing with the traditional emulsions containing surfactants, they have the following prominent advantages (1) less toxic and side effect on human bodies; (2) reducing environmental pollution; (3) strong emulsion stability, even being able to prepare emulsions having a high internal phase. Pickering emulsion reported in the current documents, however, is generally prepared from solid particles or oil phase having no biocompatibility, so that the application thereof in the biopharmaceutical field is limited. For example, the document (Zhuo Ao, Zhi Yang, Jianfang Wang, Guangzhao Zhang, To Ngai, Emulsion-Templated Liquid Core-Polymer Shell Microcapsule Formation, Langmuir, 2009, 25: 2572-2574) discloses preparing oil-in-water (o/w) Pickering emulsion with polystyrene colloidal particles, and depositing a layer shell of poly(lactide-co-glycolic acid) (also called glycolide-lactide copolymer or polylactic-hydroxyacetic copolymer or poly(D,L-lactide-co-glycolide)polymer, shortened as PLG or PLGA) on the surfaces of emulsion droplets to form a micro-capsule structure. The oil phase used therein is the mixed solution of octanol and ethyl acetate, which cannot be used in the pharmaceutical system. Moreover, polystyrene is not biodegradable. The document (Catherine P. Whitby, Li Hui Lim, Nasrin Ghouchi Eskandar, Spomenka Simovic, Clive A. Prestidge, Poly(lactic-co-glycolic acid) as a particulate emulsifier, Journal of Colloid and Interface Science, 2012, 375: 142-147) discloses preparing Pickering emulsion by using nanoparticles of high molecular materials PLGA having a better biodegradable and biocompatible (wherein the polymerization ratio of lactide to glycolide is 50:50, having a molecular weight of 40-75 kDa). The oil phase in such document includes dodecane, polydimethylsiloxane, toluene, and isopropyl myristate, wherein PLGA nanoparticles can be used for stabilizing oil-in-water (o/w) emulsion having the oil phase of dodecane and polydimethylsiloxane. However, said oil phase cannot be used as the pharmaceutical auxiliary, so that they cannot be used as the vaccine adjuvant. The document (Zengjiang Wei, Chaoyang Wang, Hao Liu, Shengwen Zou, Zhen Tong, Facile fabrication of biocompatible PLGA drug-carrying microspheres by O/W pickering emulsions, Colloids and Surfaces B: Biointerfaces, 2012, 91: 97-105) discloses preparing PLGA microspheres coated with silica nanoparticles by using silica nanoparticles as solid particles and by using dichoromethane containing PLGA as the oil phase. However, the silica and dichloromethane in such system are limited in clinical applications, and such Pickering emulsion cannot be directly applied in biopharmaceutical field. Moreover, there is no research on Pickering emulsion as a vaccine adjuvant in the current known documents or patents, and the known Pickering emulsion system is not designed or optimized according to the requirements on vaccine preparations.

**Contents of the Invention**

In view of problems existing in the prior art, one of the objects of the present invention lies in providing an oil-in-water emulsion containing no surfactants, which can be used as the vaccine adjuvant.

In order to achieve the above object, the present invention uses the following technical solution, an oil-in-water emulsion containing no surfactant, characterized in that the emulsion comprises a metabolizable oil phase, an aqueous phase and oil-water amphipathic solid particles dispersed in the aqueous phase and having biocompatibility, wherein the oil phase comprises squalene or/and tocol; the aqueous phase is any one selected from the group consisting of purified water, water for injection, glycerine aqueous solution, buffering salts aqueous solution, or the combination of at least two selected therefrom; and the solid particles have an average particle size of from 50 nm to 10μm and are

selected from the group consisting of poly(lactide-co-glycolide) particles, aluminium hydroxide particles, calcium phosphate particles, alginic acid particles coated with chitosan, calcium carbonate particles, polylactic acid particles, and chitosan particles, and the solid particles have a mass concentration of from 0.1 to 20 wt% in aqueaous phase.

In the present invention, solid particles have the oil-water amphipathy and can be absorbed on the liquid-liquid interface between the aqueous phase and the oil phase and function as stabilizing the emulsion droplets, wherein the solid particles have an average the intake amount thereof; (2) embedding, absorbing and coupling antigens with micro-nano particles may sustainable-release antigens, and prolong the cellular absorption and antigen presentation time; (3) a part of micro-nano particles (e.g. chitosan micro-nano particles having positive charges) may achieve the escape of lysosomes of antigens by proton pump effect etc,, achieve the cross presentation of antigens and promote the cellular immune response of organisms; and (4) a part of micro-nano particles may also recruit inflammatory cells so as to enhance the action between antigens and antigen-presenting cells.

Thus, the solid particles are used to replace surfactants to prepare the abovementioned oil-in-water emulsion containing no surfactant, which not only can avoid the adverse effect of surfactants on vaccine formulations, but also can obtain more comprehensive, prominent and durable immunoprotection effect by the synergistic immune actions of solid particles and oil-in-water emulsion. Such oil-in-water emulsion contains no surfactant so as to avoid the effect of surfactants on antigens. The product thereof has a better safety and stability and can be used for different inoculation routes of vaccines.

In addition, solid particles are used as the emulsion stabilizer for preparing the oil-in-water emulsion, wherein the adjuvant function of the oil-in-water emulsion can be exerted, and solid particles can also function as the immune adjuvant. The combination of solid particles and the oil-in-water emulsion may not only synergistically exert the immune enhancement and modulation, reduce the antigen amount, increase the antibody level, but also increase the diversity of the antibody type to produce antibodies having a wide scope and aiming to different types of antigens.

The oil phase is preferably one or the mixture of two selected from squalene and tocol. Squalene is a triterpene compound and has the molecular structure of isoprene having 30 carbons and 50 hydrogens, the molecular formula being 2,6,10,15,19,23-hexamethyl-2,6,10,14,18,22-tetracosahexaene, CAS:111-02-4 having the molecular mass of 410.72 and being derived from animals, plant extraction or chemical synthesis. Squalene is a metabolizable oil since it is the intermediate product of biosynthesis of cholesterol (Merck index, 10th Edition, Registration No. 8619). It is a carbons and 50 hydrogens, the molecular formula being 2,6,10,15,19,23-hexamethyl-2,6,10,14,18,22-tetracosahexaene, CAS: 111-02-4 having the molecular mass of 410.72 and being derived from animals, plant extraction or chemical synthesis. Squalene is a metabolizable oil since it is the intermediate product of biosynthesis of cholesterol (Merck index, 10th Edition, Registration No. 8619). It is a grease naturally secreted in all higher organisms, including human beings (which may be found in sebum). The emulsion containing squalene (containing surfactant) exhibits excellent immunological enhancement in animal tests and clinical tests.

Said tocol is $\alpha$-tocopherol. $\alpha$-tocopherol may have the function of enhancing the immune response in the vaccines for senile patients (e.g. patients having an age of higher than 60 or even higher). The known tocols comprise many tocopherols, such as $\alpha$, $\beta$, $\gamma$, $\delta$, $\epsilon$, $\zeta$ and the like, preferably $\alpha$-tocopherol, especially DL-$\alpha$-tocopherol. Preferably, said oil phase is incompatible with the aqueous phase, and may comprise other metabolizable oils.

In order to make the oil-in-water emulsion suitable for vaccine or pharmaceutical formulations, the oil phase in the oil-in-water emulsion of the present invention may be metabolizable oil. The term " metabolizable oil" is well known in the art. The expression "metabolizable" may be defined as "transformable by metabolism" (Medical Dictionary by Dorland, W. B. Sanders, 25th Edition (1974)).

The exemplary metabolizable oil may be any plant oil, fish oil, animal oil or synthetic oil which is non-toxic to receptors and transformable by metabolism, and comprise, but not limited to, soybean oil, miglyol 812, midchain oil, fish oil, vitamin E, vitamin E succinate, vitamin E acetate, safflower oil, corn oil, sea buckthron oil, linseed oil, peanut oil, teaseed oil, sunflower seed oil, apricot kernel oil, coix seed oil, evening primrose seed oil, sesame oil, cottonseed oil, castor oil, low-erucic acid rapeseed oil, ethyl oleate, oleic acid, ethyl linoleate, isopropyl laurate, isopropyl myristate, ethyl butyrate, ethyl lactate, caprylic triglyceride or capric triglyceride, or the combination of at least two selected therefrom. Nuts, seeds and cereal are the common sources of plant oils. and Tris buffer solution, the combination of water for injection, phosphate buffering solution and citrate buffering solution, the combination of Tris buffer solution, water for injection, phosphate buffering solution and citrate buffering solution.

Preferably, said phosphate buffering solution, citrate buffering solution or Tris buffering solution independently has a pH value of 5.0-8.1, *e.g.* 5.2, 5.4, 5.6, 5.8, 6, 6.2, 6.4, 6.6, 6.8, 7, 7.2, 7.4, 7.6, 7.8 or 8, preferably 6.0-8.0.

The aqueous phase in the oil-in-water emulsion of the present invention may comprise an univalent or polyvalent antigen, wherein the antigen comprises, but is not limited by, human antigen, non-human animal antigen, plant antigen, bacterial antigen, fungal antigen, viral antigen, parasite antigen or tumor antigen, or the combination of at least two selected therefrom. The combination comprises, e.g. the combination of human antigen and non-human animal antigen, the mixture of plant antigen and bacterial antigen, the combination of fungal antigen, viral antigen, and parasite antigen, the combination of tumor antigen, human antigen, non-human animal antigen, plant antigen, bacterial antigen and fungal antigen, the combination of viral antigen, parasite antigen, tumor antigen, human antigen, non-human animal antigen and plant antigen, the combination of bacterial antigen, fungal antigen, viral antigen, parasite antigen and tumor antigen.

The antigen may be derived from, but not limited to, chick embryo culture, cell culture, body fluid, organ or tissue from a carrier, by purification and separation, or from recombination gene expression or chemical synthesis. Preferably, the antigen comprises, but not limited to, attenuated vaccine, inactivated vaccine, split vaccine, subunit vaccine, polysaccharide conjugate vaccine, recombinant vaccine or DNA vaccine, or the combination of at least two selected therefrom. The combination above comprises, e.g. the combination of attenuated vaccine and inactivated vaccine, the combination of split vaccine and subunit vaccine, the combination of polysaccharide conjugate vaccine and recombinant vaccine, the combination of DNA vaccine and attenuated vaccine, the combination of inactivated vaccine and split vaccine, the combination of subunit vaccine, polysaccharide conjugate vaccine, recombinant vaccine and DNA vaccine.

The antigen may be a viral antigen or antigenic preparation comprising at least three influenza seasonal (during an influenza pandemic) strains, and optionally the viral antigen or antigenic preparation comprising at least one influenza virus strain related to a pandemic outbreak or having the potential of being related to a pandemic outbreak, wherein the influenza virus strains related to a pandemic outbreak or having the potential of being related to a pandemic outbreak are any one selected from the group consisting of human influenza viruses type A, B, C, including H1N1, H2N2, H3N2, H5N1, H7N7, H1N2, H9N2, H7N3, and H10N7; swine influenza viruses H1N1, H1N2, H3N1 and H3N2; dog or equine influenza viruses H7N7 and H3N8; or avian influenza viruses H5N1, H7N2, H1N7, H7N3, H13N6, H5N9, H11N6, H3N8, H9N2, H5N2, H4N8, H10N7, H2N2, H8N4, H14N5, H6N5 and H12N5, or the combination of more than one selected therefrom.

The aqueous phase in the present invention further comprises an officinal auxiliary substance, such as pH modifier or/and buffering agent etc., preferably one selected from the group consisting of sodium acetate, sodium lactate, sodium chloride, potassium chloride, calcium chloride, human serum protein, essential amino acid, non-essential amino acid, L-arginine monohydrochloride, sucrose, anhydrous D-mycose, mannitol, mannose, starch and gelatine, or the combination of at least two selected therefrom. The combination comprises, e.g. the combination of sodium acetate and sodium lactate, the combination of sodium chloride and potassium chloride, the combination of calcium chloride, human serum albumin, and essential amino acid, the combination of non-essential amino acid, L-arginine monohydrochloride, sucrose and anhydrous D-mycose, the combination of mannitol, mannose, starch and gelatine, the combination of sodium acetate, sodium lactate, sodium chloride, potassium chloride, calcium chloride and human serum albumin, the combination of essential amino acid, non-essential amino acid, L-arginine monohydrochloride, sucrose, anhydrous D-mycose, mannitol, mannose, starch and gelatine.

The oil-in-water emulsion of the present invention comprises at least one type of solid particles. The solid particles in the oil-in-water emulsion of the present invention have biocompatibility and are any one selected from the group consisting of aluminium salts, calcium salts, polysaccharides, polysaccharide derivatives or high-molecular polymers, or the mixture of at least two selected therefrom.

Preferably, the aluminium salts are aluminium hydroxide or/and aluminium phosphate. Preferably, the calcium salts are calcium phosphate or/and calcium carbonate.

Preferably, the polysaccharides are any one selected from the group consisting of chitosan, alginic acid, gelatin, starch, glucan, konjac glucomannan, heparin, pectin polysaccharides, hyaluronic acid, chondroitin sulfate, chitosan salts, alginates, gelatin salts, glucose salts, konjac glucomannan salts, heparinates, pectin polysaccharide salts, hyaluronates, chondroitin sulfate salts, or the combination of at least two selected therefrom, preferably selected from the group consisting of chitosan, alginates, gelatin, starch and glucan, or the combination of at least two selected therefrom.

Preferably, the polysaccharide derivatives are obtained by the derivation, such as quaternization, carboxymethylation, hydroxylation, alkylation, acylation, sulfonation, nitrificatioin or hylogenation, of polysaccharides, preferably by derivation, such as quaternization, carboxymethylation, hydroxylation, alkylation, acylation, sulfonation, nitrificatioin or hylogenation, of any one selected from the group consisting of chitosan, alginic acid, gelatin, starch and glucan, or the combination of at least two selected therefrom.

In the present invention, the suitable molecular weight may be determined according to the influence factors, such as the size of the antigen used therein, release rate required and the like. As for chitosan, the suitable molecular weight is about 50,000 -900,000 Daltons, preferably from 100,000 to 800,000 Daltons.

Preferably, the high-molecular polymers comprise any one selected from the group consisting of poly(a-hydroxyl acid), poly(hydroxybutyric acid), polycaprolactone, polyorthoesters, polyanhydrides, polycyanoacrylates and copolymers thereof, wherein the comonomers of the copolymers are preferably selected from the group consisting of poly(a-hydroxyl acid), poly(hydroxybutyric acid), polycaprolactone, polyorthoesters, polyanhydrides, polycyanoacrylates, or the combination of at least two selected consisting of poly(a-hydroxyl acid), poly(hydroxybutyric acid), polycaprolactone, polyorthoesters, polyanhydrides, polycyanoacrylates and copolymers thereof, wherein the comonomers of the copolymers are preferably selected from the group consisting of poly(a-hydroxyl acid), poly(hydroxybutyric acid), polycaprolactone, polyorthoesters, polyanhydrides, polycyanoacrylates, or the combination of at least two selected therefrom. The combination comprises, e.g. the combination of poly(a-hydroxyl acid) and poly(hydroxybutyric acid), the combination of polycaprolactone, polyorthoesters and polyanhydrides, the combination of polycyanoacrylates, poly(a-hydroxyl acid), poly(hydroxybutyric acid), polycaprolactone and polyorthoesters, the combination of polyanhydrides, polycyanoacrylates and poly(a-hydroxyl acid), and the combination of poly(hydroxybutyric acid), polycaprolactone, polyorthoesters, polyanhydrides and polycyanoacrylates.

Preferably, the high-molecular polymers are poly(lactide-co-glycolide) called "PLG" or "PLGA" (also called glycolide-lactide copolymers or polylactide-glycolic acid copolymers or poly(D,L-lactide-co-glycolide) polymers, also poly(lactic-co-glycolic acid), shortened as PLG or PLGA).

Other high molecular polymer not being part of the present invention are poly(a-hydroxyl acid) and copolymers thereof, preferably selected from the group consisting of poly(L-lactide), poly(D,L-lactide).

Different chain-segment molar ratios may affect the hydrophilic-hydrophobic property and degradation speed of the materials. For example, 50:50 PLGA polymers containing 50% of D,L-lactide and 50% of glycolide have a faster degradation speed. Since the ratio of lactide increases, 75:25 PLGA has a slower degradation speed.

As an exemplary of the oil-in-water emulsion containing no surfactant, the emulsion comprises a metabolizable oil phase, an aqueous phase and oil-water amphipathic solid particles dispersed in the aqueous phase and having biocompatibility. The oil phase is squalene; the aqueous phase is selected from the group consisting of purified water, water for injection, glycerine water solution, buffering salts water solution, or the combination of at least two selected therefrom; the solid particles are PLGA, and have an average particle size in a scale of from nanometer to micrometer.

The known processes can be used for obtaining these polymers having various molecular weights. The suitable molecular weight may be determined according to the influence factors, such as the size of antigens used therein, the required release rate and the like. As for poly(L- lactide) and poly(D,L- lactide), for example, the suitable molecular weight thereof is in a scale of from about 2000 to 5000 Daltons. As for PLGA, the suitable molecular weight thereof is generally from about 10000 to about 200000 Daltons, preferably from about 13,000-about 150,000 Daltons.

Preferably, the solid particles are any one selected from the group consisting of aluminium hydroxide, aluminium phosphate, calcium phosphate, calcium carbonate, chitosan, alginates, polylactic acid, or the mixture of at least two selected therfrom, further preferably selected from the group consisting of aluminium hydroxide, calcium phosphate, polylactic acid, or the mixture of at least two selected therefrom

What's called " aluminium hydroxide" is generally hydroxy aluminum oxide salts, and often at least partially crystallized. Hydroxy alumina oxide is represented with the molecular formula AIO(OH). It is different from other aluminium compounds, e.g. $Al(OH)_3$, in the infrared spectrum, and especially there are a absorption band at 1070 cm$^{-1}$ and a strong shoulder peak at 3090-3100 cm$^{-1}$. Aluminium hydroxide exhibits a from 0.5 to 20 $\mu$m (e.g. about 5-10 $\mu$m). At a pH of 7.4, aluminium phosphate has an absorbing ability of 0.7-1.5 mg protein per mg of $Al^{3+}$.

In various calcium salts, calcium phosphate is preferred as the solid particles in the present invention. Various adjuvant forms of calcium phosphate have been reported, and the present invention may use any of these forms. The adjuvants may form needle-like particles having a size of about 10 nm×150 nm, and sheets having irregular shapes and a diameter of about 20-30 nm. There are documents disclosing calcium phosphate particles ("CAP") having a diameter of 300-400 nm (nanoparticles), a spherical shape and a smooth surface. The aforesaid calcium phosphates all can achieve the present invention. The solid particles in the present invention may have a shape of sphere, rod, cambiform, disc, cube, peanut or amorphous shape and the like. The morphology of solid particles may be smooth surface, porous surface, internal multicavity, hollow or monocular. Those skilled in the art may optimize and screen according to the oil or water phase used therein and antigen properties by limited processes to obtain the oil-in-water emulsion satisfying the application requirements.

The solid particles in the oil-in-water emulsion of the present invention have the oil-water amphipathy, and can be stably dispersed at the interface of the oil-water two phases to stabilize the emulsion. As for different oil-water systems, solid particles having different hydrophilic-hydrophobic properties may be chosen to stabilize the emulsion, or the surfaces of solid particles may be hydrophlically or hydrophobically modified, coated or graft-modified to obtain a suitable hydrophilic-hydrophobic property (or particle wettability, generally represented by oil-water-solid contact angle $\theta_{ow}$).

Preferably, the surfaces or interiors of solid particles may absorb, couple or embed the functional substances, such as targeting substances, fluorescence indicators, isotope labelling substances, environmental response substances, cytokines, antibodies or immunomodulators. The environmental response substances are selected from those with the pH-sensitive, heat sensitive or bioactivator sensitivegroups.

The surfaces or interiors of the solid particles may also absorb, couple or embed antigens as the delivery system of antigens, which may increase the stability of antigens, promote the antigen intake of antigen-presenting cells and increase the immune response.

The solid particles may be prepared by many methods. For example, poly(lactide-co-glycolide) solid particles may be prepared by many methods, such as solvent evaporation, solvent extraction, deposition and the like. Chitosan solid particles may be prepared by the single emulsification method (forming the water-in-oil emulsion) in combination with the chemical crosslinking method (e.g. crosslinking with glutaraldehyde dissolved in the oil phase), or by spray-drying or deposition method. After preparation, solid particles may be kept in the aqueous solution or buffering solution, or be lyophilized for standby.

Preferably, said solid particles have an average particle size between 1 nm and 10 $\mu$m, *e.g.* 5 nm, 10 nm, 50 nm, 100 nm, 200 nm, 300 nm, 400 nm, 500 nm, 600 nm, 700 nm, 800 nm, 900 nm, 1 $\mu$m, 2 $\mu$m, 3 $\mu$m, 4 $\mu$m, 5 $\mu$m, 6 $\mu$m, 7 $\mu$m, 8 $\mu$m or 9 $\mu$m, preferably between 10 nm and 5 $\mu$m.

Preferably, said solid particles have a particle size distribution coefficient span value of less than 1.0.

Preferably, said solid particles have a mass concentration of between 0.1 and 20 wt%, *e.g.* 0.5 wt%, 1 wt%, 2 wt%, 3 wt%, 4 wt%, 5 wt%, 6 wt%, 7 wt%, 8 wt%, 9 wt%, 10 wt%, 11 wt%, 12 wt%, 13 wt%, 14 wt%, 15 wt%, 16 wt%, 17 wt%, 18 wt% or 19 wt%, preferably between 0.5 and 10wt%, further preferably between 1 and 8 wt%, in the aqueous phase. The mass concentration of said solid particles in the aqueous phase is the specific value of the mass of solid particles to the mass sum of solid particles and aqueous phase.

Preferably, the volume ratio of the oil phase and aqueous phase in the oil-in-water emulsion of the present invention is between 1:100 and 9:1, *e.g.* 1:90, 1:80, 1:70, 1:60, 1:50, 1:40, 1:30, 1:20, 1:10, 1:1, 2:1, 3:1, 4:1, 5:1, 6:1, 7:1 or 8:1, preferably between 1:50 to 1:2.

Preferably, the emulsion droplets in the oil-in-water emulsion have an average particle size between 50 nm and 300 $\mu$m, *e.g.* 100 nm, 200 nm, 300 nm, 400 nm, 500 nm, 600 nm, 800 nm, 1 $\mu$m, 10 $\mu$m, 30 $\mu$m, 50 $\mu$m, 80 $\mu$m, 110 $\mu$m, 140 $\mu$m, 170 $\mu$m, 200 $\mu$m, 230 $\mu$m, 260 $\mu$m or 290 $\mu$m, preferably between 100 nm and 100 $\mu$m.

The oil-in-water emulsion of the present invention may further comprise pharmaceutical additives, e.g. any one selected from diluents, stabilizers and preservatives, or the combination of at least two selected therefrom.

The oil-in-water emulsion of the present invention may further comprise, but not limited to, the following adjuvants, pattern recognition receptors (e.g. stimulants of Toll-like receptors, RIG-1 and NOD-like receptors (NLR), e.g. oligonucleotides containing CpG sequence motif, double-stranded RNA, oligonucleotides containing Palindrome sequence(s), or oligonucleotides containing poly(dG) sequences), mineral salts (*e.g.* alums, alums combining with monophosphoryllipid (MPL) A of intestinal bacteria (e.g. *E.Coli,* Minnesota Salmonella, Salmonella typhimurium or Shigella bacteria), or alums respectively and specifically combining with MPL® (AS04) and the bacteria MPLA mentioned above), MPL, saponin (e.g. QS-21, Quil-A, iscoMs and iscomatrix™), lipidosome and lipidosome preparation (e.g. As01), synthetic or specially prepared microparticles and microcarriers (e.g. bacteria-derived OMV of N.gonorrheae, Chlamydia trachomatis and other bacteria), polysaccharides (e.g. chitosan), specially modified or prepared peptides (e.g. Muramyl dipeptide), aminoalkylglucosaminide 4-phosphate (*e.g.* RC529) or protein (*e.g.* bacterial toxin or toxin fragment), selectable pathogen-associated molecular pattern (PAMPS), small molecular immunopotentiator (SMIP), cytokines and chemotactic factors. Cytokines include, but not limited to granulocyte-macrophage colony stimulating factor (GM-CSF), interferon (e.g. interferon-$\alpha$ (IFN-a), interferon-$\beta$ (IFN-$\beta$), interferon-$\gamma$ (IFN-y) etc.), interleukin (e.g. interleukin-1$\alpha$ (IL-1$\alpha$), interleukin-1$\beta$ (IL-1$\beta$), interleukin-2 (IL-2), interleukin-4 (IL-4), interleukin-7 (IL-7), interleukin-12 (IL-12), interleukin-15 (IL-15), interleukin-18 (IL-18)), fetal liver tyrosine kinase 3 ligand (Fit3L) or tumor necrosis factor-$\alpha$ (TNF-$\alpha$) and the like.

The oil-in-water emulsion of the present invention may be prepared by various methods.

Specifically, the oil-in-water emulsion of the present invention may be prepared by the following methods, but not limited by the following preparation methods.

The oil-in-water emulsion of the present invention may be prepared by first dispersing the solid particles in the aqueous phase, and then mixing the oil phase and the aqueous phase. The solid particles may be dispersed by vibration, stirring, ultrasonic dispersion and the like, to achieve better dispersion of solid particles in the aqueous phase. As long as better dispersion of particles in the aqueous phase can be achieved, the dispersion modes will not have a significant effect on the properties of the oil-in-water emulsion. The suitable dispersion modes and specific operating parameters may be chosen according to the properties of the aqueous phase and solid particles, and the experimental facility conditions. The mixing of the oil phase and the aqueous phase may be achieved by microfluidization, homogenization, ultrasound, two-syringe emulsification, spraying, microjet, microchannel emulsification, membrane emulsification, stirring, vibration, inversion or hand-cranking mixing. According to different requirements, the mixing modes may be preferably micro fluidization, microchannel emulsification or membrane emulsification to obtain the emulsion having a homogeneous particle size distribution. Microjet, two-syringe emulsification, homogenization, stirring or vibration may be preferably used for large-scale preparation. It should be made clear that different modes of mixing oil phase and aqueous phase will necessarily affect the particle size of droplets in the emulsion, the emulsion stability and the like, and will affect the final immune or administration effects. As mentioned in one of the examples of the present invention, the membrane emulsification, as compared with mechanical stirring, may be used to obtain the emulsion having a more homogeneous particle size distribution and smaller emulsion droplets, which may lead to higher level of antibodies. Thus, the suitable mixing modes and specific operating parameters shall be determined according to the influence factors, such as the properties of the oil phase, aqueous phase and solid particles, the particle size range of the emulsion droplets to be prepared.

The solid particles, oil phase and aqueous phase in the oil-in-water emulsion of the present invention may be respectively and independently packed, and temporarily mixed before application according to the abovementioned preparation methods, or two or three of them may be mixed beforehand according to the abovementioned preparation methods.

The oil-in-water emulsion of the present invention may be sterilized by moist heat sterilization or filtration sterilization. When the particle size of the particles used is less than 220 nm, the filtration sterilization is preferably used.

When the oil-in-water emulsion comprises antigens, said oil-in-water emulsion may be independently packed with antigens, temporarily mixed before immunization, or inoculated to the same site within a shorter time interval (generally within 1 h, including 1 h), or according to the abovementioned preparation methods, mixed first then packed, and directly applied during the immunization.

Another object of the present invention lies in providing an immunogenic composition comprising no surfactant, comprising (1) an antigen or antigen composition, and (2) an adjuvant composition consisting of the oil-in-water emulsion above.

Another object of the present invention lies in providing the use of the oil-in-water emulsion above as the vaccine adjuvant, drug delivery or controlled-releasecarrier. In the present invention, the immunization or administration modes comprise intravenous injection, vertebra caval injection, intramuscular injection, subcutaneous injection, intracutaneous injection, spraying or inhalation via respiratory tract, intraperitoneal injection, nasal administration, ocular administration, oral administration, rectal administration, vaginal administration, topical administration or transdermal administration. The oil-in-water emulsion as the vaccine adjuvant may be used for human beings, domestic animals, poultries and aquatic products.

As compared with the prior art, the present invention has the following beneficial effects.

The present invention first combines solid particles with oil emulsion formulation (oil-in-water emulsion) to prepare the oil-in-water emulsion containing no surfactant, which is applied in the field of developing vaccine adjuvants. The addition of solid particles can not only increase the formulation biocompatibility, avoid the adverse effect of surfactants on human bodies, animals or vaccines, but also more effectively stimulate immune cells, motivate the immune modulating function. Meanwhile, the solid particles have the properties which are easy to control, and may be surface-modified or coated. Or solid particles having different properties (e.g. composition, morphology, structure, particle size and the like) may be chosen to function by different immunological enhancement mechanism, and embed, absorb or couple antigens and be used as the delivery carrier of antigens. The antigen controlled-release capability thereof is used to modulate the immune response, and they can also be used for various immunization modes.

The main immunological enhancement mechanisms of the oil-in-water emulsion of the present invention comprise (1) if said particles therein absorbing or embedding antigens, such oil-in-water emulsion may delay the release rate of antigens, protect antigens from hydrolysis, postpone the in vivo residence time of antigens, and be advantageous to the production of antibodies having high affinity; (2) the particles may activate macrophages, and promote the interactions of macrophages with T and β cells, so as to specifically enhance the stimulation to lymphocytes; (3) said particles absorbing or embedding antigens may increase the surface area of antigens to make antigens easy to be phagocytosed by macrophages; (4) the oil-in-water emulsion may also induce slight inflammatory response at the injection sites, recruit the inflammatory cells, stimulate the secretion of inflammatory factors, and activate the immunoreaction; (5) some special particles, e.g. chitosan particles having pH sensitivity, may achieve the escape of antigen lysosome and increase the cellular immunologic response after absorbing or embedding antigens.

In addition, the oil-in-water emulsion of the present invention may also be used for drug delivery or controlled-release carrier. By dispersing the liposoluble drugs, fluorescence indicators or other bioactive substances in the oil phase, or embedding or absorbing drugs, fluorescence indicators or other bioactive substances on the surface of particles, controlled release of drugs are achieved. By coupling or embedding the targeting substances (e.g. Fe3O4, folic acid, mannose, etc.) on the surface of or in the interior of particles, the oil-in-water containing such particles may also be used for targeting-delivery of drugs, fluorescence indicators or other bioactive substances.

Thus, the oil-in-water emulsion of the present invention can be used as not only the immunologic adjuvants of vaccines, but also the delivery or controlled-release carriers of drugs or other bioactive substances.

## Description of the drawings

Fig.1 shows the schematic diagram of the oil-in-water Pickering emulsion.
Fig.2 shows the particle size distribution of PLGA particles prepared in Example 1.
Fig.3 shows the stereoscan photograph of PLGA particles prepared in Example 1.
Fig.4 shows the light microscope photograph of the Pickering emulsion prepared in Example 1 (20x magnification).
Fig.5 shows the photographs of the Pickering emulsion prepared in Example 1 before and after centrifugation (1 shows before centrifugation, and 2 shows after centrifugation).
Fig.6 shows the particle size distribution of aluminium hydroxide particles prepared in Example 2.

Fig.7 shows the light microscope photograph of the Pickering emulsion prepared in Example 2 (20x magnification)
Fig.8 shows the photograph of the Pickering emulsion prepared in Example 4.

## Embodiments

The technical solution of the present invention is further explained by combining with the figures and the embodiments.

The present invention may be achieved by the following examples, but not limited to the materials, apparatus, ratio, composition, method, steps in the examples. It shall be understood that the examples and embodiments discussed herein are used only for explanation, and anybody who is familiar to such field may put forward improvements and changes which fall within the spiritual nature and scope of the present application and within the scope of the attached claims.

List of the materials

| Name | Specification | Manufacturer |
|---|---|---|
| PLGA | LA:GA including (90:10, 80:20, 75:25, 70:30, 60:40, 50: 50, 40:60, 30:70, 20: 80, 10:90); the molecular weight includes 13000, 59000, 78000, 119000, 134000, 150000, 200000 Daltons | Jinan Daigang Biotechnology ltd., Co |
| PLA | the molecular weight being 73000 | Jinan Daigang Biotechnology ltd., Co |
| Acetone | Analytically pure | Beijing Chemical Works |
| Petroleum ether | Analytically pure | Beijing Chemical Works |
| Acetonitrile | Analytically pure | West Long Chemical Co., Ltd. |
| Absolute ethyl alcohol | Analytically pure | Beijing Chemical Works |
| Polyvinyl alcohol (PVA) | Viscosity of 5.0 mPa·s, alcohylysis degree of 99% | Shanxi Sanwei Group Co., Ltd. |
| Squalene | Purity ≥98% | Sigma Aldrich |
| $\alpha$-tocopherol | Chromatographically pure | Sigma Aldrich |
| Triton X-100 | Analytically pure | Beijing Chemical Reagent Company |
| n-butanol | Analytically pure | Beijing Chemical Works |
| Cyclohexane | Analytically pure | Beijing Chemical Works |
| Aqueous ammonia | Analytically pure | Sinopharm Chemical Reagent Co., Ltd. |
| $AlCl_3$ | Analytically pure | Beijing Chemical Reagent Company |

(continued)

| Name | Specification | Manufacturer |
|---|---|---|
| Tween 80 | Analytically pure | Beijing Chemical Reagent Company |
| PEG 6000 | Analytically pure | Sigma Aldrich |
| Trishydroxymethylaminomethane (Tris) | Analytically pure | Sigma Aldrich |
| Concentrated hydrchloric acid | Analytically | Beijing Chemical Reagent Company |
| Calcium chloride | Analytically | Sinopharm Chemical Reagent Co. |
| Sodium hydrogen phosphate | Analytically | Sinopharm Chemical Reagent Co. |
| Sodium dihydrogen phosphate | Analytically | Sinopharm Chemical Reagent Co. |
| Magnesium chloride | Analytically | Sinopharm Chemical Reagent Co. |
| Glacial acetic acid | Analytically | Sinopharm Chemical Reagent Co. |
| Citric acid | Analytically | Sinopharm Chemical Reagent Co. |
| Span 80 | Analytically | Sinopharm Chemical Reagent Co., Ltd. |
| Monomethoxy polyethylene glycol-lactic copolymer (PELA) | The molar ratio of mPEG:PLA being 1:19, having an average molecular weight of 40 kDa | Jinan Daigang Biotechnology ltd., Co |
| Sodium dodecyl sulfate (SDS) | Analytically pure | Sinopharm Chemical Reagent Co., Ltd. |
| Chitosan | The molecular weight including 50000, 100000, 500000, 800000, 900000 Daltons, deacetylation degree including 80%, 85%, 90% and 95% | Golden-Shell Pharmaceutical Co., Ltd. |
| Alginic acid | The molecular weight of 450000-550000 Daltons | Acros Organics |
| Glycerophosphate | Pharmaceutical grade | Sigma Aldrich |
| PO-500 | Food grade | Sakamoto Yakuhin Kogyo Co., Ltd |

(continued)

| Name | Specification | Manufacturer |
|---|---|---|
| Dichloromethane | Analytically | Sinopharm Chemical Reagent Co., Ltd. |
| Calcium acetate | Analytically | Sinopharm Chemical Reagent Co. |
| Trisodium citrate | Analytically | Sinopharm Chemical Reagent Co. |
| Sodium carbonate | Analytically | Sinopharm Chemical Reagent Co. |
| Glycerol | Analytically | Sinopharm Chemical Reagent Co. |
| Ethyl acetate | Analytically | Sinopharm Chemical Reagent Co. |
| Sodium chloride | Analytically | Sinopharm Chemical Reagent Co. |

Apparatus

| Name | Specification | Manufacturer |
|---|---|---|
| Electronic scales | TB-214 | Beijing Sartorius Instrument Systems, Inc. |
| Injection syringe promoting pump | PDH-2000 | Harvard Apparatus |
| Centrifugal machine | Avanti-J-E | Beckman-Coulter |
| Ultrasonic washing unit | 25-12 | Ningbo Xinzhi Biotechnology Co., Ltd. |
| Magnetic stirring apparatus | Color Squid | IKA |
| Lyophilizer | Alpha 2-4 LD | CHRIRT |
| Scanning electron microscope | JSM-6700F | JEOL |
| Refrigerator | MDF-382E(N) | SANYO |
| Optical microscope | Olympus BX51 | Olympus |
| Homogenizer | T18 Basic | IKA |
| Pipette | 200 μL, 1 mL, 5 mL, 10 mL | Eppendorf |
| Laser particle analyzer | Mastersizer 2000E | Malvern |
| Zeta potential and granulometer | ZetaPlus | Brookhaven Instuments Corporation |
| Ultrasonic cell crusher | S-450D | Branson Inc. |
| Vortex oscillator | Vortex-Genie 2 | Scientific Industries, Inc. |

(continued)

| Name | Specification | Manufacturer |
|---|---|---|
| Microplate reader | Infinite M200 | Tecan |

Property characterization:

Measurement of particle size distribution of particles or emulsion droplets

The particle size distribution of micrometer-sized particles or emulsion droplets is measured with laser particle size analyzer. The specific measuring steps comprise adding 5 mg of micrometer-sized particles into 50 mL of deionized water, ultrasounding for 5 min to homogeneously disperse, or taking 50 mL of the emulsion, adding the particle suspension or emulsion into the sample cell and measuring with laser particle size analyzer (Malvern Instruments, United Kingdom Coulter Co., USA).

The particle size distribution of nanometer-sized particles or emulsion droplets is measured with Zeta potential and granulometer. The specific measuring steps comprise adding 1 mg of nanometer-sized particles into 10 mL of deionized water, ultrasounding for 5 min to homogeneously disperse, or taking 2 mL of the emulsion, adding the particle suspension or emulsion into the sample cell, which is then placed ino the Zeta Potential Analyzer (Brookhaven Instruments Corporation) for measurement.

The homogeneity of the particles or droplets is represented by the particle size distribution coefficient (Span) value. Span is calculated by the following formula, wherein the smaller the value is, the more homogeneous the particle size is:

$$\text{Span} = (d_{90} - d_{10})/d_{50} \quad (1)$$

Wherein $d_{10}$, $d_{50}$ and $d_{90}$ are respectively the particle sizes when the accumulative volumes of particles are 10%, 50% and 90%.

The morphological observation of the particles was made by the scanning electron microscopy (SEM): weighing 1 mg of particles, adding them to 10 mL of deionized water, ultrasounding for 5 min to homogeneously disperse; drawing 1 mL of the suspension, dripping it on a sheet of aluminium foil, homogeneously spreading out it on the aluminium foil, naturally drying; pasting the sheet of aluminium foil onto a sample stage with the conducting resin, metal spraying under vacuum conditions (choosing suitable metal spraying conditions according to the sample properties), and then observing with the SEM.

The morphological observation of the emulsion droplets was made by the optical microscope: drawing a small amount of the oil-in-water emulsion, dripping it onto the glass slide, and observing it under the optical microscope.

The stability of the emulsion was measured by centrifugation: taking 5 mL of the oil-in-water emulsion, adding it into 15 mL of the centrifugal tube, then centrifugalizing 10 min under the centrifugal force of 2000 g, and observing the delamination.

The measurement of the embedding rate and loading capacity of antigens or drugs in particles:

Accurately weighing 10 mg of drug-loading particle lyophilized powder, completely degrading the microspheres by the suitable methods (e.g. degrading the microspheres by adding NaOH or acetonitrile for the polylactic acid microspheres, degrading the microspheres by adding diluted hydrochloric acid for chitosan microspheres); neutralizing the degradation solution with NaOH or hydrochloric acid after the particles are completely degraded; making pH be 7 and then metering the volume to be 2 mL.

The antigen or drug content was measured by BCA kit or micro-BCA kit or other suitable measuring method. The antigen or drug embedding rate was calculated by the following formula:

Embedding rate=(measured antigen or drug amount in particles/antigen or drug addition amount during the actual preparation)×100%

The loading capacity of antigens or drugs on particles was calculated by the following formula:

$$\text{Loading capacity} = (\text{measured antigen or drug amount in particles/measured mass of particles})$$

The measurement of the adsorption rate and loading capacity of antigens or drugs absorbed on particles:

Taking the suspension of particles adsorbing antigens or drugs, centrifugalizing and obtaining the supernatant (choosing suitable centrifugal conditions according to the size and density of particles), measuring the concentration of antigens or drugs in the supernatant so as to indirectly calculate the amount of antigens or drugs adsorbed onto the surface of particles. The amount of antigens or drugs was measured by BCA kit or micro-BCA kit or other suitable measuring method. The adsorbing rate of antigens or drugs was calculated by the following formula:

$$\text{Adsorbing rate} = (\text{the antigen or drug concentration before adsorption - the antigen or drug concentration in the supernatant after adsorption})/ \text{the antigen or drug concentration before adsorption} \times 100\%$$

The loading capacity of antigens or drugs on the particles was calculated by the following formula:

$$\text{Loading capacity} = (\text{measured amount of antigens or drugs on particles/measured mass of particles})$$

Measurement by animal experiment:

Balb/c mice used in the experiment were provided by Vital River Company. The immunization steps are basically as follows: randomly grouping the mice, conducting the experiment with more than 6 mice in each group, grouping and immunizing the mice according to the specific description in the examples; drawing 200 $\mu$L of blood before inoculation, centrifugalizing it for 5 min at 12000 rpm immediately to separate the serum out, measuring the IgG antibody level, taking the IgG antibody level at this time as the initial value, and then immunizing the mice; after immunization, drawing blood from the eye or tail tip of the mice regularly, 200 $\mu$L each time, measuring the IgG antibody level; performing the second immunization after two weeks; killing the mice after 35 days, drawing the blood, measuring the IgG antibody level (further measuring the hemagglutination titer (HI) for the influenza vaccines); harvesting and cultivating the spleen cells of mice, and measuring the secretion of IL-4 and IFN-$\gamma$ cytokines in the supernatant of the spleen cell culture of mice with the enzyme-linked immunosorbent assay (ELISA).

**Example 1** Preparation of the oil-in-water emulsion by using poly-PLGA particles Preparation of PLGA particles by nanoprecipitation method

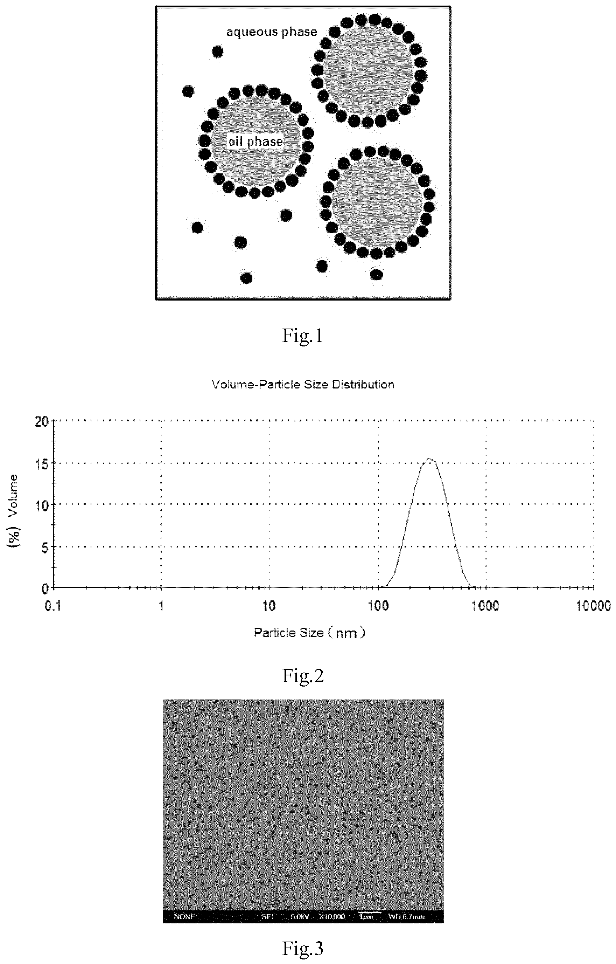

Accurately weighing 0.30 g of PLGA (LA:GA being 50:50, the molecular weight being 110000 Daltons) with an electronic scale, dissolving them into 1 mL of acetone, dropwise adding such solution with No.9 syringe needle at a rate of 1 drop per second into 20 mL of the aqueous solution (containing 1 wt.% of PVA (having an alcoholysis degree of 99% and a viscosity of 5.0 mPa·s), magnetic stirring with a stir speed of 500 rpm), stirring at 25°C overnight, centrifugalizing the resulting solution for 20 min at 20000 g, discarding the supernatant, adding 5 mL of deionized water into the precipitate, dispersing by ultrasounding, centrifugalizing the solution for 5 min at 20000 g, discarding the supernatant, lyophilizing the precipitate to obtain the PLGA particles, placing the particles in the refrigerator at 4°C, wherein the resultant PLGA particles had an average particle size of 226 nm, and a Span of 0.535. By observing with the SEM, it was found that the resultant particles had smooth surfaces and ordered sphere shapes. The particle size distribution of particles is shown in Fig.2, and the morphology thereof is shown in Fig.3.

Preparation of the oil-in-water emulsion

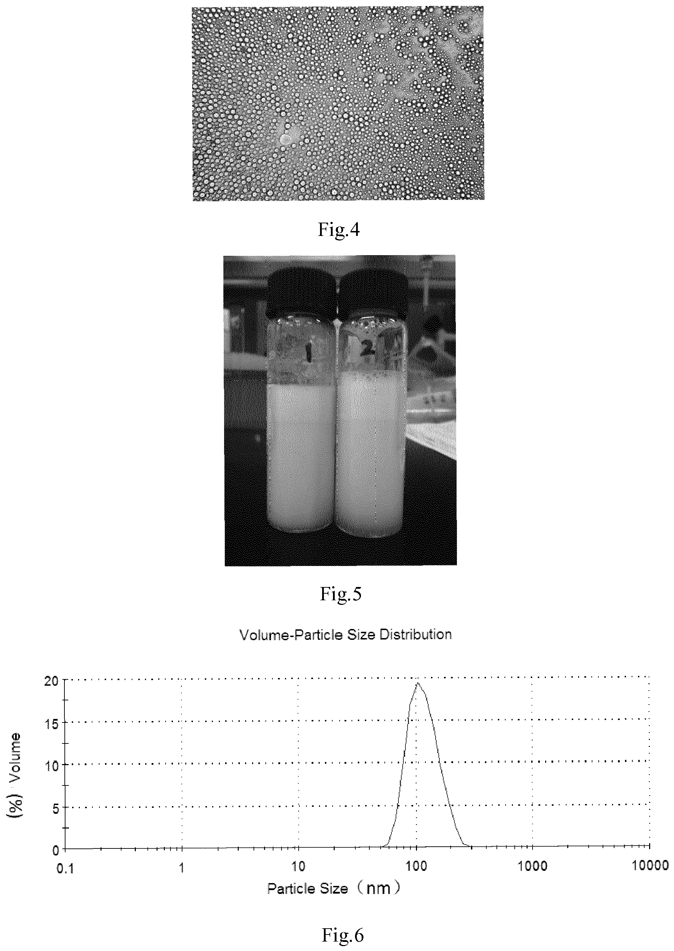

Accurately weighing 0.50 g of PLGA particles with an electronic scale, adding them into 4 mL of deionized water, ultrasounding for 1 min to homogeneously disperse to obtain an aqueous phase suspension containing particles dispersed therein, wherein the aqueous phase had a pH of 6.5; drawing 1 mL of squalene with pipette and adding the same to the aforesaid aqueous phase suspension, preparing the oil-in-water emulsion by homogeneous emulsification (15000 rpm, 3 min), wherein the emulsion droplets had a better dispersibility and ordered sphere shapes, had an average particle size of 18.9$\mu$m and a Span of 0.895. The stability of the emulsion was measured by centrifugation. The appearance of the prepared oil-in-water emulsion has no difference from that of the uncentrifugalized emulsion, and there is no oil phase separated out at the superstratum. The light microscope of the emulsion is shown in Fig.4, and the photographs of the emulsion before and after centrifugation are shown in Fig.5

Other PLA particles may also be used for preparing the oil-in-water emulsion, and the preparation steps are similar to those in Example 1. The specific processing parameters and results are shown in Table 1.

Table 1

| Formula | | | | | | | | | | | | | | Processing technology | | Results | |
|---|---|---|---|---|---|---|---|---|---|---|---|---|---|---|---|---|---|
| Solid particles | | | | | Aqueous phase | | | Oil phase | | Volume ratio of oil phase to aqueous phase | Mass concentration of particles in the aqueous phase (wt. %) | Dispersing mode of particles in the aqueous phase | Mixing mode of the aqueous phase and oil phase | | | Properties of the emulsion | |
| materials | LA GA | Molecular weight (Dalton) | Particle size (nm) | Span | Ingredients | pH | Other additives | Ingredients | Other additives | | | | | | | Average particle size ($\mu$m) | Span |
| I | 10:90 | 13000 | 202 | 0.81 | Deionized water | 6.7 | 0.1 mol/L NaCl | squalene | 0.01 mg/mL retinoic acid | 1:10 | 1 | Magnetic stirring (250 rpm, 2 min) | Homogeneity (6000 rpm, 3 min) | | | 30.2 | 0.892 |
| | 20:80 | 13000 | 5320 | 0.92 | Deionized water | 6.7 | 0.1 mol/L KCl | squalene | Soybean oil (volume ratio to squalene being 1:1) | 1:5 | 0.1 | Ultrasounding dispersion (200 W, 30 s) | Double push syringe emulsification | | | 297 | 0.769 |

(continued)

| Formula | | | | | | | | | | | Processing technology | | Results | |
|---|---|---|---|---|---|---|---|---|---|---|---|---|---|---|
| Solid particles | | | | | Aqueous phase | | | Oil phase | | Volume ratio of oil phase to aqueous phase | Mass concentration of particles in the aqueous phase (wt. %) | Dispersing mode of particles in the aqueous phase | Mixing mode of the aqueous phase and oil phase | Properties of the emulsion | |
| materials | LA GA | Molecular weight (Dalton) | Particle size (nm) | Span | Ingredients | pH | Other additives | Ingredients | Other additives | | | | | Average particle size (μm) | Span |
| PLGA | 30:70 | 59000 | 10 | 0.65 | Tris buffering solution | 7.3 | 0.05 mol/L NaCl | α-tocopherol | Peanut oil (volume ratio to α-tocopherol being 1:1) | 1:100 | 0.5 | Mechanically stirring (250 rpm, 2 min) | Micro-Fluidization homogeneity (12000 psi) | 0.102 | 0.532 |
| | 40:60 | 78000 | 1.5 | 0.44 | PBS buffering solution | 7.4 | 0.01 mol/L sucrose+150 μg/mL H5N1 avian influenza split vaccine | α-tocopherol | Corn oil (volume ratio to α-tocopherol being 1:2) | 1:50 | 10 | Homogeneity (2500 rpm, 50s) | Rapid membrane emulsification (membrane pore size being 1.4 μm, transmembrane pressure being 3 MPa, transmembrane 5 times) | 0.051 | 0.362 |

EP 3 015 114 B1

18

(continued)

| Formula | | | | | | | | | | | | | Processing technology | | Results | |
|---|---|---|---|---|---|---|---|---|---|---|---|---|---|---|---|---|
| Solid particles | | | | | Aqueous phase | | | Oil phase | | Volume ratio of oil phase to aqueous phase | Mass concentration of particles in the aqueous phase (wt. %) | | Dispersing mode of particles in the aqueous phase | Mixing mode of the aqueous phase and oil phase | Properties of the emulsion | |
| materials | LA GA | Molecular weight (Dalton) | Particle size (nm) | Span | Ingredients | pH | Other additives | Ingredients | Other additives | | | | | | Average particle size (μm) | Span |
| | 50:50 | 13000 | 205 | 0.79 | Deionized water | 6.7 | 0.1 mol/L NaCl | squalene | - | 1:10 | 1 | | Magnetic stirring (250 rpm, 2 min) | Homogeneity (6000 rpm, 3 min) | 10.5 | 0.682 |
| | 60:40 | 119000 | 70 | 0.64 | Deionized water | 6.7 | 0.01 mol/L saponin | Mixture of squalene to α-tocopherol (volume ratio of 1:1) | 0.01 mg/mL retinoic acid | 1:10 | 20 | | Mechanically stirring (150 rpm, 3 min) | Mechanically stirring (500rpm, 5 min) | 24.5 | 0.831 |

EP 3 015 114 B1

| | Formula | | | | | | | | | | | Processing technology | | Results | |
|---|---|---|---|---|---|---|---|---|---|---|---|---|---|---|---|
| Solid particles | | | | | Aqueous phase | | | Oil phase | | Volume ratio of oil phase to aqueous phase | Mass concentration of particles in the aqueous phase (wt. %) | Dispersing mode of particles in the aqueous phase | Mixing mode of the aqueous phase and oil phase | Properties of the emulsion | |
| materials | LA:GA | Molecular weight (Dalton) | Particle size (nm) | Span | Ingredients | pH | Other additives | Ingredients | Other additives | | | | | Average particle size ($\mu$m) | Span |
| | 70:30 | 134000 | 7000 | 0.32 | Citric acid buffering solution | 6.0 | - | Mixture of squalene to $\alpha$-tocopherol (volume ratio of 2:1) | 0.01 mg/mL taxol | 9:1 | 15 | Vortex oscillation (3 min) | Homogeneity (2000 rpm, 3 min) | 264.3 | 0.765 |
| | 75:25 | 13000 | 220 | 0.77 | Deionized water | 6.7 | 0.1 mol/L NaCl | squalene | - | 1:10 | 1 | Magnetic stirring (250 rpm, 2 min) | Homogeneity (6000 rpm, 3 min) | 32.4 | 0.543 |

EP 3 015 114 B1

(continued)

| | | Formula | | | | | | | | | | Processing technology | | Results | |
|---|---|---|---|---|---|---|---|---|---|---|---|---|---|---|---|
| | | Solid particles | | | Aqueous phase | | | Oil phase | | Vol ume rati o of oil pha se to aqu eou s pha se | Mas s con centrati on of part icle s in the aqu eou s pha se (wt. %) | Dispe rsing mode of particl es in the aqueo us phase | Mixi ng mod e of the aque ous phas e and oil phas e | Properties of the emulsion | |
| m a t er ia l s | LA GA | Molecu larweight (Dalton ) | Partic le size (nm) | Sp an | Ingr edie nts | pH | Other additi ves | Ingre dients | Other additive s | | | | | Ave rag e part icle size ($\mu$ m) | Spa n |
| | 80: 20 | 150000 | 916 | 0.9 2 | Dei oniz ed wat er | 6.7 | 200 $\mu$g/m L H1N1 Influe nza A whole virus inactivated vacci ne | squale ne | - | 1:2 | 3 | Vertic al mixin g (180 rpm, 2 min) | Micr oflui dies (T-ch annel , the dispe rsion phas e havi ng a pipe orific e diam eter of 50$\mu$ m and a flow rate of 0.03 mL/min, the conti nuou s phas e havi ng a pipe orific e diam eter of 0.4 mm and a flow rate of 9.4 mL/ min) | 197 . 6 | 0.2 31 |
| | 90: 10 | 200000 | 220 | 0.1 5 | Dei oniz ed wat er | 6.7 | 0.001 mol/L gelati n | squale ne | - | 5:1 | 5 | Ultras oundi ng (60 W, 30 s) | Mag netic stirri ng (500r pm, 5 min) | 5.4 | |

| Formula | | | | | | | | | | | Processing technology | | Results | |
|---|---|---|---|---|---|---|---|---|---|---|---|---|---|---|---|
| Solid particles | | | | | Aqueous phase | | | Oil phase | | Volume ratio of oil phase to aqueous phase | Mass concentration of particles in the aqueous phase (wt. %) | Dispersing mode of particles in the aqueous phase | Mixing mode of the aqueous phase and oil phase | Properties of the emulsion | |
| materials | LA GA | Molecular weight (Dalton) | Particle size (nm) | Span | Ingredients | pH | Other additives | Ingredients | Other additives | | | | | Average particle size (μm) | Span |
| PLA | - | 73000 | 9954 | 0.68 | PBS buffering solution | 7.4 | 0.001 μg/mL MPL | squalene | - | 4:1 | 7 | Magnetic stirring (250 rpm, 2 min) | Vortex oscillation (10 min) | 267.2 | 0.825 |

**Example 2** Preparation of the oil-in-water emulsion by using aluminium hydroxide particles

Preparation of aluminium hydroxide particles by microemulsion method

Triton X-100, n-butanol and cyclohexane were mixed in a volume ratio of 1:0.5:20 and magnetically stirred (800 rpm, 5 min) to obtain the oil phase. The syringe propulsion pump was used to dropwise add 1 mol/L of AlCl$_3$ solution (2 mL) to the oil phase (20 mL) under magnetically stirring (500 rpm) to obtain the reversed-phase microemulsion of aluminium chloride. Ammonia was dropwise added by the syringe propulsion pump to the aforesaid reversed-phase microemulsion at a rate of 0.5 mL/min to keep the reaction system to have a pH of higher than 10.0. After 2 h of the reaction, 10 mL of acetone was added to demulsify. The supernatant was removed after centrifugation (15000 g, 5 min). Absolute ethyl alcohol was used to repeatedly wash three times, and deionized water was used to wash once to finally obtain aluminium hydroxide particles after lyophilization. The particles were kept in the refrigerator at 4°C. The particles had an average particle size of 120.5 nm and a Span of 0.486. The morphology of the prepared particles was close to sphere, and the particle size distribution of the particles is shown in Fig.6.

Preparation of the oil-in-water emulsion:

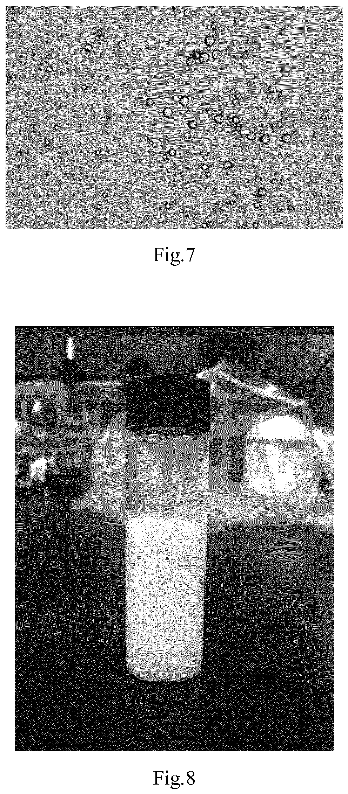

Accurately weighing 0.72 g of aluminium hydroxide particles with an electronic scale, adding them to 40 mL of phosphate buffering solution, ultrasounding for 1 min to homogeneously disperse to obtain an aqueous phase suspension containing particles dispersed therein, wherein the aqueous phase had a pH of 7.4; drawing 2 mL of squalene with pipette and adding the same to the aforesaid aqueous phase suspension, preparing the oil-in-water emulsion by homogeneous emulsification (20000 rpm, 5 min), wherein the emulsion droplets had a better dispersibility and ordered sphere shapes, had an average particle size of 7.4 $\mu$m and a Span of 0.696. The appearance of the prepared oil-in-water emulsion has no difference from that of the uncentrifugalized emulsion, and there is no oil phase separated out at the superstratum. The light microscope of the emulsion is shown in Fig.7.

**Example 3** Preparation of the oil-in-water emulsion by using calcium phosphate particles

Preparation of hollow calcium phosphate particles by the template method

3.0 g Tween 80, 0.25 g PEG 6000, 3.0 mL 0.5mol/L of Tris-HCl (pH8.0) and 1.5 mL of deionized water were mixed and homogeneously stirred, ultrasounded for 20 min (10W) to form non-ionic surfactant vesicae. 3.55 mL of CaCl$_2$ (0.175 mol/L) solution was dropwise added and stirred for 0.5 h. Then 3.55 mL of Na$_2$HPO$_4$ (0.175 mol/L) solution was added to obtain a suspension of sodium phosphate particles which was then stabilized by adding 0.83 mL MgCl$_2$ solution (0.075 mmol/L). After stirring for another 2 h, centrifugal washing, vacuum drying, hollow calcium phosphate particles were obtained. The particles had an average particle size of 210 nm, a shell thickness of 30-40 nm and a Span of 0.349. The prepared particles are hollow spheres.

Preparation of the oil-in-water emulsion:

Accurately weighing 1.00 g of hollow calcium phosphate particles with an electronic scale, adding them to 20 mL of citric acid buffering solution, ultrasounding for 2 min to homogeneously disperse to obtain an aqueous phase suspension containing particles dispersed therein, wherein the aqueous phase had a pH of 6.0; drawing 2 mL of squalene with pipette and adding the same to the aforesaid aqueous phase suspension, preparing the oil-in-water emulsion by the magnetic stirring emulsification (500 rpm, 10 min), wherein the emulsion droplets had a better dispersibility and ordered sphere shapes, had an average particle size of 10.2 $\mu$m and a Span of 0.696. The appearance of the prepared oil-in-water emulsion has no difference from that of the uncentrifugalized emulsion, and there is no oil phase separated out at the superstratum.

**Example 4** Preparation of the oil-in-water emulsion by using alginic acid particles coated with chitosan

Preparation of alginic acid particles coated with chitosan by the rapid membrane emulsification

Firstly, petroleum ether (having a boiling range of 60-90°C) was mixed with liquid paraffin in a volume ratio of 2:1, and 4 wt. % of emulsifier Span 80 was added to the mixed organic phase. The aforesaid mixture was used as the oil phase, and the aqueous phase was the aqueous sodium alginate solution (1.0 wt.%). 2 mL of the aqueous phase and 60 mL of the oil phase were emulsified under the homogenizing action (3600 rpm, 1 min) to form a pre-emulsion. Then the pre-emulsion was added to the rapid membrane emulsification storage rank, passed through the SPG membrane (having a membrane pore size of 1.4μm) under a nitrogen pressure (1 MPa) to obtain a more homogeneous emulsion after 5 cycles. CaCl$_2$ solution (5 mol/L, 12 mL) was used as the curing agent and dispersed in the oil phase (24 mL) under the action of ultrasounding (120 W, 1 min) to form a miniemulsion. Such miniemulsion was mixed with the abovementioned homogeneous emulsion and stirred for 5 h (250 rpm) in the water bath at 37°C to cure the emulsion and obtain colloidal particles. The particles were washed respectively with petroleum ether, ethanol and water for three times to obtain the alginic acid particles. The coating steps of alginic acid particles with chitosan comprise first dispersing 1 g of alginic acid particles in 0.7 wt.% of chitosan acetic acid solution (20 mL, wherein the chitosan molecular weight was 800,000 Daltons, and the deacetylation degree was 90%), stirring for 1 h (200 rpm), washing the particles with acetic buffering solution (pH 4 and pH 5.5) and deionized water to obtain the alginic acid particles with chitosan coatings. The particles may further be multi-coated, wherein the steps comprise dispersing the coated colloidal particles (1 g) to 0.5 wt.% of the aqueous sodium alginate solution (20 mL), stirring for 1 h (200 rpm), washing with deionized water once, then dispersing the particles to 0.7 wt.% of chitosan acetic solution (20 mL) and stirring for 1 h (200 rpm). Then the particles were washed with acetic buffering solution (pH 4 and pH 5.5) and deionized water to obtain the double-coated chitosan-alginic acid particles. The aforesaid steps were repeated for many times to obtain the multi-coated chitosan alginic acid particles. In the present invention, the alginic acid particles were triple-coated, wherein the particles had an average particle size of 457 nm and a Span of 0.839. The prepared particles had rough surfaces and regular sphere shapes.

Preparation of the oil-in-water emulsion:

Accurately weighing 1.22 g of alginic acid particles coated with chitosan with an electronic scale, adding them to 10 mL of phosphate buffering solution, ultrasounding for 1 min to homogeneously disperse to obtain an aqueous phase suspension containing particles dispersed therein, wherein the aqueous phase had a pH of 8.0; drawing 2 mL of squalene with pipette and adding the same to the aforesaid aqueous phase suspension, preparing the oil-in-water emulsion by vortex oscillation (10 min) emulsification, wherein the emulsion droplets had a better dispersibility and ordered sphere shapes, had an average particle size of 10.2 μm and a Span of 0.741. After centrifugation of the prepared oil-in-water emulsion, about 1 cm of oil phase was separated out at the superstratum. The photograph of the emulsion is shown in Fig.8.

**Example 5** Preparation of the oil-in-water emulsion by using monomethoxypolyethylene glycol-co-lactide copolymer (PELA) porous particles

Preparation of PELA porous particles by the phase separation-solvent removal method

100 mg PELA (mPEG:PLA molar ratio being 1:19, the average molecular weight being 40 kDa) was dissolved in 7.5 mL of acetone, and 7.5 mL of absolute ethyl alcohol was added thereto. The aforesaid solution was dropwise added (one drop/s) to deionized water (90 mL, containing 10 g/L SDS) under rapidly stirring (750 rpm). Afterthe addition is completed, stirr for another 24 h (750 rpm). Deionized water was used to centrifugal wash for 5 times, and the precipitate was suspended and kept in 10 mL of deionized water to form the PELA particle suspension.

200 μL of dichloromethane was added to 3 mL of deionized water, and then homogeneously mixed in the ultrasonic cell crusher (400 W, 60s) to serve as the swelling agent. 2 mL of the aforesaid PELA particle suspension was added into the swelling agent, under magnetic-stirring (250 rpm), swelled for 30 min, stood for 1 h and rapidly placed into liquid nitrogen for rapid freezing, kept at -20°C to evaporate the organic solvent to obtain the porous PELA particles having an average particle size of 78.55 nm and a Span of 0.331 and being the surface-porous spheres.

Preparation of the oil-in-water emulsion:

Accurately weighing 0.351 g of PELA particles with an electronic scale, adding them to 10 mL of water for injection, ultrasounding for 1 min to homogeneously disperse to obtain an aqueous phase suspension containing particles dispersed therein, wherein the aqueous phase had a pH of 7.0; the oil phase being 2 mL of α-tocopherol, and preparing the oil-in-water emulsion by the forward tapered microfluidic emulsification (the taper end having a diameter ranging from 20 to 40 μm,the oil phase having a flow rate of 500-600 μL/h, the aqueous phase having a flow rate of 1000-1200 μL/h), wherein the emulsion droplets had a better dispersibility and ordered sphere shapes, had an average particle size of 70 μm and a Span of 0.096. The appearance of the prepared oil-in-water emulsion has no difference from that of the uncentrifugalized emulsion, and there is no oil phase separated out at the superstratum.

**Example 6** Preparation of the oil-in-water emulsion with the peanut-like calcium carbonate particles

Preparation of the peanut-like calcium carbonate particles by the liquid phase direct mixing and precipitation method

Calcium acetate and trisodium citrate were weighed by an electronic scale and dissolved in 200 mL of distilled water (wherein trisodium citrate had a mass concentration of 10 wt.% and 30 wt.% respectively). 10 wt.% of the aqueous sodium carbonate solution (50 mL) was added into such solution, reacted for 3 h while stirring (300 rpm), filtered, washed respectively with distilled water and absolute ethyl alcohol and precipitated three times, dried at 70°C to obtain the peanut-like calcium carbonate particles having a length of 7.2 $\mu$m and a major-minor axis ratio of 2:1 and having a peanut-like morphology.

Preparation of the oil-in-water emulsion

3.15 g of calcium carbonate particles was accurately weighed by an electronic scale, and added into 40 mL of phosphate buffering solution, ultrasounded for 1 min to homogeneously disperse to obtain the aqueous phase suspension in which the particles were dispersed, wherein the aqueous phase had a pH of 7.4. 2 mL of squalene was drawn by the pipette and added to the aforesaid aqueous phase suspension. The oil-in-water emulsion was prepared by the rapid membrane emulsification method (Shirasu Porous Glass (SPG) microporous membrane having a pore size of 50.2 $\mu$m, the transmembrane pressure being 200 Kpa, and passing through the membrane three times). The emulsion droplets had a better dispersibility and regular sphere shapes, an average particle size of 45.10 $\mu$m and a Span of 0.726. After centrifugation of the prepared oil-in-water emulsion, 0.5 cm of the oil phase was separated out at the superstratum.

**Example 7** Preparation of the oil-in-water emulsion with the polylactic acid (PLA) particles embedded with antigens

Preparation of the PLA particles embedded with HBsAg by the rapid membrane emulsification in combination with the multiple emulsion-solvent removal method

200 mg of PLA was dissolved in 0.4 mL of ethyl acetate. 0.4 mL of 5% (w/v) of HBsAg was added for pre-emulsification with the ultrasonic cell crusher (with a power of 12% for 15s) under the ice-water bath. Then the pre-emulsion was poured to 200 mL of the aqueous solution containing 1.0 wt.% of PVA (the aqueous external phase) for pre-multiple-emulsification (300 rpm, 50s) by magnetically stirring. After pre-multiple-emulsification, the resultant pre-multiple-emulsion was poured into the storage tank of the rapid membrane emulsification apparatus, and pressed through the SPG membrane (having a pore size of 5.2 $\mu$m) with nitrogen having a pressure of 300 KPa to obtain the multiple emulsion. The multiple emulsion was poured into 800 mL of 0.9 wt.% of NaCl solution (the curing solution), and magnetically stirred at 500 rpm for 10 min to cure the microspheres. The cured microspheres were centrifugalized (4000 r/min, 5 min), washed with deionized water for three times and finally lyophilized to obtain the product. The particles had an average size of 2.32 $\mu$m and a Span of 0.496, and were spheres with smooth surfaces. The antigens had an embedding rate of 90%, and the particles had an antigen load of 0.09 mg antigen/g microspheres.

Preparation of the oil-in-water emulsion

7.22 g of PLA particles were accurately weighed by an electronic scale, and added into 40 mL of water for injection, vortex-oscillated for 5 min for homogeneous dispersion to obtain the aqueous phase suspension in which the particles are dispersed, wherein the aqueous phase had a pH of 7.0. 2 mL of $\alpha$-tocopherol was drawn with the pipette and added to the aforesaid aqueous phase suspension. The oil-in-water emulsion was prepared by the micro jet emulsification (12,000 psi). The emulsion droplets had a better dispersibility and regular sphere shapes, and an average particle size of 42.39 $\mu$m and a Span of 0.742. The appearance of the prepared oil-in-water emulsion has no difference from that of the uncentrifugalized emulsion, and there is no oil phase separated out at the superstratum.

**Example 8** Preparation of the oil-in-water emulsion by using chitosan particles

Preparation of chitosan particles by the rapid membrane emulsification in combination with the temperature curing method

Formulation of the aqueous phase:

A certain amount of chitosan (having a molecular weight of 50,000 Daltons and a deacetylation degree of 80%) was accurately weighed and dissolved in 9 mL of the acetic acid solution (0.1 mol/L), magnetically stirred to completely dissolve to obtain the chitosan acetic acid solution. Additionally, a certain amount of sodium glycerophosphate was dissolved in 1 mL of deionized water. The chitosan acetic acid solution and sodium glycerophosphate solution were respectively kept at 4°C for 10 min, and subsequently the sodium glycerophosphate solution was slowly and dropwise added to the chitosan acetic acid solution, magnetically stirred (300 rpm, 10 min) and homogeneously mixed. Such solution was centrifugalized at 20,000 rpm to remove the insoluble impurities and to keep the supernatant as the aqueous phase for standby. The chitosan had a concentration of 3.5 wt.% in the aqueous phase, and sodium glycerophosphate had a concentration of 10.0 wt.% in the aqueous phase.

Preparation of the oil phase:

The oil-soluble emulsifier PO-500 was added to 60 mL of the mixture (having a volume ratio of 5:7) of liquid paraffin and petroleum ether (having a boiling range of 60-90°C), wherein PO-500 had a concentration of 4 wt.% in the oil phase and was stirred to completely dissolve and kept at 4°C for 10 min as the oil phase.

Preparation of the emulsion

At 4°C, 2mL of the aqueous phase and 50 mL of the oil phase were mixed and emulsified by the homogeneity emulsifier at 6,000 rpm for 1 min to form the pre-emulsion. The pre-emulsion was rapidly poured into the pre-emulsion storage tank of the rapid membrane emulsification apparatus and passed through the SPG microporous membrane (having a membrane pore size of 2.8 $\mu$m) at a nitrogen pressure of 5.0 Mpa to obtain the W/O-type emulsion having a homogeneous particle size. The resultant emulsion was used as the pre-emulsion, re-passed through the SPG microporous membrane at a nitrogen pressure of 5.0 Mpa, and repeatedly emulsified 5 times to finally obtain the W/O-type emulsion having a homogeneous particle size. The emulsification process took about 10 min. After completing the emulsification, the W/O-type emulsion was placed in a water bath at 35°C, mechanically stirred (200 rpm) and cured for 1 h. After curing, the emulsion was centrifugalized at 10,000 rpm, washed with petroleum ether, ethanol and deionized water in turn to obtain the chitosan particles having an average diameter of 870 nm and a Span of 0.487. The particles were spheroidal and had loosen and porous surfaces.

Preparation of the oil-in-water emulsion

1.00 g of the chitosan particles were accurately weighed by an electronic scale, and added into 20 mL of the PBS buffering solution, ultrasounded for 1 min to homogeneously disperse to obtain the aqueous suspension in which the particles are dispersed, wherein the aqueous phase had a pH of 8.1. 2 mL of $\alpha$-tocopherol was drawn with the pipette and added to the aforesaid aqueous phase suspension. The oil-in-water emulsion was prepared by the vortex-oscillation (10 min). The emulsion droplets had a better dispersibility and regular sphere shapes, and an average particle size of 18.10 $\mu$m and a Span of 0.935. The appearance of the prepared oil-in-water emulsion has no difference from that of the uncentrifugalized emulsion, and there is no oil phase separated out at the superstratum.

Other chitosan materials can also be used for preparing the particles and the following oil-in-water emulsion. The preparation steps are similar to those in Example 8, and their differences lie in using chitosans having different molecular weights and deacetylation degrees, and changing the composition of oil phase and aqueous phase and the preparation processes. The specific processing parameters and results are shown in Table 2. Under the same deacetylation degree conditions, the less the molecular weight is, the lower the viscosity is; the smaller the particle size of the particles prepared under the same membrane emulsification conditions is, the smaller the particle size of the prepared emulsion is. Under the same molecular weight conditions, the higher the deacetylation degree is, the lower the viscosity is; the smaller the particle size of the particles prepared under the same membrane emulsification conditions is, the smaller the particle size of the prepared emulsion is.

Table 2

| | | | | | | | | | | | | | | | |
|---|---|---|---|---|---|---|---|---|---|---|---|---|---|---|---|
| | | | | Formula | | | | | | | | Preparation Technology | | Results | |
| | | Solid particles | | | | Aqueous phase | | | Oil phase | | Vol ume ratio of oil phas e to aqu eous phas e | Mass concen tration of particle s in the aqueou s phase (wt.%) | Dispe rsing mode of partic les in the aqueo us phase | Mixing mode of aqueou s phase and oil phase | Properties of the emulsion |
| Mate rials | Deacet ylation degree | Mole cular weig ht (Dalt on) | Part icle size (nm ) | Sp an | Ingre dients | p H | Othe r addit ives | Ingre dients | Othe r addit ives | | | | | Ave rage parti cle size (μm ) | Sp an |
| Chit osan | 80% | 5000 0 | 870 | 0.4 87 | PBS buffer ing soluti on | 7 . 4 | 0.1 mol/ L NaC 1 | squal ene | - | 1:10 | 5 | Magn etic stirrin g (250 rpm, 5 min) | Homog eneity (6000 rpm, 3 min) | 14.3 | 0.6 89 |
| | 80% | 1000 00 | 932 | 0.4 58 | PBS buffer ing soluti on | 7 . 4 | 0.1 mol/ L NaC I | squal ene | - | 1:10 | 5 | Magn etic stirrin g (250 rpm, 5 min) | Homog eneity (6000 rpm, 3 min) | 18.5 | 0.7 71 |
| | 80% | 5000 00 | 112 5 | 0.5 32 | PBS buffer ing soluti on | 7 . 4 | 0.1 mol/ L NaC I | squal ene | - | 1:10 | 5 | Magn etic stirrin g (250 rpm, 5 min) | Homog eneity (6000 rpm, 3 min) | 21.2 | 0.8 35 |
| | 80% | 8000 00 | 135 4 | 0.4 43 | PBS buffer ing soluti on | 7 . 4 | 0.1 mol/ L NaC 1 | squal ene | - | 1:10 | 5 | Magn etic stirrin g (250 rpm, 5 min) | Homog eneity (6000 rpm, 3 min) | 30.3 | 0.9 89 |

(continued)

| Materials | Solid particles | | | | Formula | | | | | Volume ratio of oil phase to aqueous phase | Mass concentration of particles in the aqueous phase (wt.%) | Preparation Technology | | Results | |
| --- | --- | --- | --- | --- | --- | --- | --- | --- | --- | --- | --- | --- | --- | --- | --- |
| | | | | | Aqueous phase | | | Oil phase | | | | Dispersing mode of particles in the aqueous phase | Mixing mode of aqueous phase and oil phase | Properties of the emulsion | |
| | Deacetylation degree | Molecular weight (Dalton) | Particle size (nm) | Span | Ingredients | pH | Other additives | Ingredients | Other additives | | | | | Average particle size (μm) | Span |
| | 80% | 900000 | 1578 | 0.546 | PBS buffering solution | 7.4 | 0.1 mol/L NaCl | squalene | - | 1:10 | 5 | Magnetic stirring (250 rpm, 5 min) | Homogeneity (6000 rpm, 3 min) | 34.2 | 0.996 |
| | 85% | 800000 | 1277 | 0.654 | PBS buffering solution | 7.4 | 0.1 mol/L NaCl | squalene | - | 1:10 | 5 | Magnetic stirring (250 rpm, 5 min) | Homogeneity (6000 rpm, 3 min) | 25.6 | 0.768 |
| | 90% | 800000 | 982 | 0.435 | PBS buffering solution | 7.4 | 0.1 mol/L NaCl | squalene | - | 1:10 | 5 | Magnetic stirring (250 rpm, 5 min) | Homogeneity (6000 rpm, 3 min) | 20.3 | 0.641 |
| | 95% | 800000 | 964 | 0.335 | PBS buffering solution | 7.4 | 0.1 mol/L NaCl | squalene | - | 1:10 | 5 | Magnetic stirring (250 rpm, 5 min) | Homogeneity (6000 rpm, 3 min) | 17.9 | 0.632 |

**Example 9** Preparation of the oil-in-water emulsion by using the chitosan particles adsorbing antigens

The method of preparing particles is the same as that in Example 8, except that the chitosan particles adsorb H5N1 avian influenza split vaccines:

1 g of the prepared chitosan particles were accurately weighed, and added to 10 mL of the PBS buffering solution containing H5N1 avian influenza split vaccines (having an HA concentration of 150 $\mu$g/mL), oscillated (120 rpm, 24 h) at 4°C, centrifugalized at 10,000 rpm, washed three times with deionized water to obtain the chitosan particles adsorbing H5N1 avian influenza split vaccines, wherein the antigens had an adsorbing rate of 60%, and the antigen load of particles was 900 $\mu$gHA/g particles.

Preparation of the oil-in-water emulsion

1.00 g of the chitosan particles adsorbing antigens were accurately weighed by an electronic scale, and added into 20 mL of the PBS buffering solution, ultrasounded for 1 min to homogeneously disperse to obtain the aqueous phase suspension in which the particles are dispersed, wherein the aqueous phase had a pH of 8.1. 2 mL of $\alpha$-tocopherol was drawn with the pipette and added to the aforesaid aqueous phase suspension. The oil-in-water emulsion was prepared by the vortex-oscillation (10 min). The emulsion droplets had a better dispersibility and regular sphere shapes, and an average particle size of 19.70 $\mu$m and a Span of 0.941. The appearance of the prepared oil-in-water emulsion has no difference from that of the uncentrifugalized emulsion, and there is no oil phase separated out at the superstratum.

**Example 10** Safety evaluation of the oil-in-water emulsion

1. Vascular stimulation test

Those oil-in-water emulsions prepared in Examples 1 and 2 were respectively injected by ear vein to rabbits, once per day and consecutively for three days. The results showed that there was no significant change at the administration sites of rabbit ear veins by visual observation. The microscope inspection of the tissue pathological section showed that the blood vessel at 1 cm away from the administration site, and the blood vessel endothelium at 5 cm away from the administration site were continuous and intact, and there was no proliferation or swelling; there was no inflammatory cells infiltration or necrosis in the perivascular tissue; there was no thrombogenesis inside the lumen. There showed that the products had no significant stimulation against the vein vessel of rabbit ears.

2. Hemolysis and agglomeration test

By the conventional *in vitro* test tube (visual observation), those prepared in Examples 1 and 2 were respectively mixed with 2% erythrocytes suspension. No hemolysis and erythrocyte agglomeration occur within 3 hours.

3. Muscle stimulation test

Those prepared in Examples 1 and 2 were administered by injection to quadriceps femoris of rabbits, 1 mL per side. After 48 hours, the administered sites were taken, visually observed and histopathologically examined. The results showed that the products had no irritation against quadriceps femoris of rabbits

**Example 11** Effect of the mixing mode of the aqueous phase and the oil phase in the oil-in-water emulsion on the production of the antibody titer

Preparation of PLGA particles according to the method in Example 1

Preparation of the oil-in-water emulsion

2 g of PLGA particles were accurately weighed by an electronic scale, and added into 40 mL of the citric acid buffering solution, ultrasounding for 5 min for homogeneous dispersion to obtain the aqueous phase suspension in which the particles are dispersed, wherein the aqueous phase had a pH of 6.0. 2 mL of squalene was drawn with the pipette and added to the aforesaid aqueous phase suspension. The aforesaid mixed solution was equally divided into two parts which were respectively emulsified by the rapid membrane emulsification (having a membrane pore size of 40.2$\mu$m and a transmembrane pressure of 500 Kpa, and passing through the membrane three times) and the mechanical stirring (500 rpm, 5 min), to prepare the oil-in-water emulsion. The emulsion droplets had a better dispersibility and regular sphere shapes, wherein the emulsion droplets in the emulsion prepared by the rapid membrane emulsification had an average particle size of 32.20 μm and a Span of 0.372; the emulsion droplets in the emulsion prepared by the mechanical stirring had an average particle size of 45.32 μm and a Span of 0.839. The appearance of the prepared oil-in-water emulsion has no difference from that of the uncentrifugalized emulsion, and there is no oil phase separated out at the superstratum.

The oil-in-water emulsions prepared above were respectively mixed with H5N1 avian influenza split vaccine (both having the hemagglutinin (HA) content of 4.5 μg/100 μL) (ultrasonic (10 W, 3 min) mixing the emulsion and vaccine in a volume ratio of 1:1). The split vaccine having the same hemagglutinin content was used as thecontrol. Balb/c mouse was injected at the left hind leg muscle, revaccinated after two weeks, and killed after 35 days. The results are shown in Table 3. As compared with the mechanical stirring group, the rapid membrane emulsification group can have better adjuvant effect, and the tested animal produced *in vivo* a high level of IgG and HI titer. There was prominent difference between the adjuvant group and the single antigen injection group.

Table 3

| Antigen type | Adjuvant type | Preparation mode of emulsion | Serum IgG level | HI level |
|---|---|---|---|---|
| Split vaccine | Example 1 | Rapid membrane emulsification | 480000 | 2560 |
| Split vaccine | Example 1 | Mechanical stirring | 320000 | 640 |
| Split vaccine | None | None | 62000 | 32 |

Example 12 Effect of the mixing mode of the oil-in-water emulsion and antigen on the production of antibody titer

The oil-in-water emulsions prepared according to the method in Example 11 were respectively mixed with H5N1 avian influenza inactivated whole virus vaccine and split vaccine (both having a hemagglutinin (HA) content of 4.5 μg/100 μL) (the emulsion and vaccine having a mixing ratio of 1:1, being mixed in different modes, including oscillation (5 min), ultrasounding (10 W, 3 min) and homogenizing (10,000 rpm, lmin)). Balb/c mice were respectively intramuscularly injected to the left hind leg, revaccinated after two weeks, and killed after 35 days. The results are shown in Table 4, wherein the oil-in-water emulsions both can have better adjuvant effect. The tested animals produced *in vivo* a high level of IgG and HI titer, significantly different from those of the single antigen injection group, wherein the injection group of the oil-in-water emulsion mixed by the homogenizing method had a higher antibody level.

Table 4

| Antigen type | Adjuvant type | Mixing mode | Serum IgG level | HI level |
|---|---|---|---|---|
| Inactivated whole virus vaccine | Example 1 | Oscillation | 320000 | 720 |
| Inactivated whole virus vaccine | Example 1 | Ultrasound | 510000 | 2580 |
| Inactivated whole virus vaccine | Example 1 | Homogenizing | 600000 | 3600 |
| Inactivated whole virus vaccine | None | None | 80000 | 64 |
| Split vaccine | Example 1 | Oscillation | 300000 | 640 |
| Split vaccine | Example 1 | Ultrasound | 480000 | 2560 |
| Split vaccine | Example 1 | Homogenizing | 580000 | 3200 |
| Split vaccine | None | None | 62000 | 32 |

Example 13 Effect of the oil-in-water emulsion and the antigen inoculation mode on the adjuvant effect

The oil-in-water emulsions prepared according to the method in Example 11 were respectively mixed with the recombinant HBsAg Vaccine (recombinant Hansenula vaccine, having an antigen concentration of 1.9 mg/mL) (the emulsion and vaccine having a mixing ratio of 1:1, being homogeneously mixed (10,000 rpm, lmin)). Balb/c mice were respectively intramuscularly, subcutaneously and intraperitoneally injected with the aforesaid vaccine compositions, revaccinated after two weeks, and killed after 35 days. The results are shown in Table 5, wherein the intramuscular injection group shows a higher humoral antibody level (IgG) and cytokine secretion level (IL-4 and IFN-γ).

Table 5

| Antigen type | Adjuvant type | Inoculation mode | Serum IgG level | IL-4 | IFN-γ |
|---|---|---|---|---|---|
| Hepatitis B vaccine | Example 1 | Intramuscular injection | 256000 | 32 | 11240 |
| Hepatitis B vaccine | Example 1 | Subcutaneous injection | 184000 | 28 | 10020 |
| Hepatitis B vaccine | Example 1 | Intraperitoneal injection | 102400 | 18 | 8000 |
| Hepatitis B vaccine | None | Intramuscular injection | 20480 | 5 | 2000 |
| Hepatitis B vaccine | None | Subcutaneous injection | 14560 | 4 | 1540 |
| Hepatitis B vaccine | None | Intraperitoneal injection | 10240 | 2 | 1000 |

**Example 14** Comparison of the adjuvant effects of the oil-in-water emulsions, aluminium adjuvant and MF59 adjuvant

The oil-in-water emulsions prepared in Example 11, aluminium hydroxide adjuvant and MF59 were respectively mixed with H1N1 influenza inactivated whole virus vaccine in a volume ratio of 1:1 to obtain various vaccine adjuvant compositions, wherein the hemagglutinin concentration in each composition was 15 μg/mL, 0.5 mL per dose. The inactivated whole virus vaccine having a hemagglutinin concentration of 15 μg/mL was taken as control. Balb/c mice were intramuscularly injected with equal volumes of the aforesaid vaccine adjuvant compositions and the control vaccine for inoculation, revaccinated after two weeks, and killed after 35 days. The immune results are shown in Table 6. The results show that the oil-in-water emulsions of the present invention may prominently increase the humoral immune response level and cellular immune response level against the influenza inactivated whole virus vaccine. The oil-in-water emulsions have an enhanced effect on the humoral immunity, which is equal to that of MF59 group and higher than that of the aluminium hydroxide adjuvant group. All three of them are higher than the vaccine without adjuvants. In terms of cellular immunity, the oil-in-water emulsion groups are better than the MF59 group and the aluminium hydroxide adjuvant group, which is mainly due to the fact that the particles in the oil-in-water emulsions may promote the phagocytosis of antigens by the antigen presenting cells, activate the lymphocytes, stimulate the differentiation thereof and secrete cytokines.

Table 6

| Antigen type | Adjuvant type | Serum IgG level | HI level | IL-4 | IFN-γ |
|---|---|---|---|---|---|
| H1N1 Influenza A inactivated whole virus vaccine | Example 1 | 512000 | 2560 | 30 | 12000 |
| H1N1 Influenza A inactivated whole virus vaccine | MF59 | 501200 | 1280 | 20 | 8000 |
| H1N1 Influenza A inactivated whole virus vaccine | Aluminium hydroxide adjuvant | 242400 | 640 | 15 | 4000 |
| H1N1 Influenza A inactivated whole virus vaccine | None | 92400 | 64 | 3 | 1000 |

**Example 15** Effect of the addition amount of the oil-in-water emulsion on the immune effect

The oil-in-water emulsions prepared according to the method in Example 11 and EV71 HFMD inactivated virus vaccine were mixed in different ratios, *e.g.* 2:1, 1:1, 1:2 and 1:4, to compose various vaccine adjuvant compositions, wherein the antigen concentration in each composition was 0.05 mg/mL, and each dose was 0.2 mL. The aforesaid vaccine adjuvant compositions were inoculated to Balb/c mice, and the second immunization was made after two weeks. The immunization results are shown in Table 7. The results show that the increase of the adjuvant amount in the vaccine adjuvant composition may enhance the immune response level produced by quantitative antigens, and the adjuvant dose has positive correlation with the adjuvant potency.

Table 7

| Antigen type | Adjuvant type | Mixing ratio of adjuvant to antigens | Serum IgG level |
|---|---|---|---|
| EV71 HFMD inactivated virus vaccine | Example 1 | 2 : 1 | 204800 |
| EV71 HFMD inactivated virus vaccine | Example 1 | 1 : 1 | 145000 |
| EV71 HFMD inactivated virus vaccine | Example 1 | 1 : 2 | 81400 |
| EV71 HFMD inactivated virus vaccine | Example 1 | 1 : 4 | 51200 |
| EV71 HFMD inactivated virus vaccine | None | 无 | 10240 |

**Example 16** Effect of vaccines having different antigen concentrations on the immune effect

The oil-in-water emulsions prepared according to the method in Example 11 and H5N1 influenza inactivated whole virus vaccine composed the vaccine adjuvant compositions having the hemagglutinin concentrations of 37.5, 75 and 150 $\mu$g/mL, wherein each dose was 0.1 mL. The aforesaid two vaccine adjuvant compositions and two adjuvant-free vaccines having the same hemagglutinin concentration were intramuscularly inoculated to Balb/c mice. The second immunization was made after two weeks, and the mice were killed after 35 days. The immunization results are shown in Table 8. The results show that the oil-in-water emulsion of the present invention has a better adjuvant effect on H5N1 influenza inactivated whole virus vaccine. As compared with the adjuvant-free vaccine having the same hemagglutinin concentration, the adjuvant vaccine can significantly increase the *in vivo* immune response strength of mice.

Table 8

| Antigen type | Adjuvant type | Antigen concentration | 28-day serum IgG level | 35-day serum IgG level | HI level |
|---|---|---|---|---|---|
| H5N1 Influenza inactivated whole virus vaccine | Example 1 | 37.5 $\mu$g/mL | 514200 | 543800 | 3400 |
| H5N1 Influenza inactivated whole virus vaccine | Example 1 | 75 $\mu$g/mL | 620000 | 640200 | 3600 |
| H5N1 Influenza inactivated whole virus vaccine | Example 1 | 150 $\mu$g/mL | 624800 | 640200 | 3600 |
| H5N1 Influenza inactivated whole virus vaccine | None | 37.5 $\mu$g/mL | 72240 | 80480 | 100 |
| H5N1 Influenza inactivated whole virus vaccine | None | 75 $\mu$g/mL | 78560 | 84600 | 120 |
| H5N1 Influenza inactivated whole virus vaccine | None | 1501 $\mu$g/mL | 78600 | 85000 | 120 |

**Example 17** The adjuvant effect of the oil-in-water emulsion for nasal mucosa inoculation

The oil-in-water emulsions prepared according to the method in Example 11 and H7N9 influenza split vaccine composed the vaccine adjuvant compositions, each having a hemagglutinin concentration of 150 $\mu$g/mL, wherein each dose was 0.03 mL. The aforesaid vaccine adjuvant compositions and the adjuvant-free vaccine having the same hemagglutinin concentration were intranasally inoculated to Balb/c mice. The second immunization was made after two weeks, and the mice were killed after 35 days. The immunization results are shown in Table 9. The results show that the oil-in-water emulsion of the present invention has a better adjuvant effect on H7N9 influenza split vaccine. As compared with the adjuvant-free vaccine having the same hemagglutinin concentration, the adjuvant vaccine can significantly increase the immune response strength of mouse serum and mucosa.

Table 9

| Antigen type | Adjuvant type | 28-day serum IgG level | 35-day serum IgG level | HI level | IgA level in nasal mucosa lavage fluid |
|---|---|---|---|---|---|
| H7N9 Influenza split vaccine | Example 1 | 204800 | 102400 | 100 | 2024 |
| H7N9 Influenza split vaccine | None | 8400 | 7000 | 16 | 128 |

**Example 18** Partial enhancing mechanism of the oil-in-water emulsion as the immune adjuvant (local inflammation-causing effect)

In order to observe the immunoreaction induced by three different emulsion vaccine adjuvants of MF59, AS03 and the oil-in-water emulsion of the present invention (prepared according to Example 11), chicken ovalbumin (OVA) as the antigen was mixed respectively with three emulsion vaccine adjuvants to prepare the adjuvant vaccine compositions. The aforesaid adjuvant vaccine compositions were inoculated by intramuscularly injecting to Balb/c mouse right hind leg. The mice were killed at different times. The tissues of the injection sites of mice were sliced, and the sliced tissues were dyed by H&E and TUNEL to observation the *in vivo* inflammatory reactions induced by three emulsion vaccine adjuvants. The local tissue slice observation showed that three emulsion vaccine adjuvants all can result in a certain inflammatory reaction and recruit the inflammatory cells. However, the inflammation was slight, and the inflammatory response would fade away after seven days. There was a better biocompatibility.

**Example 19** Partial enhancing mechanism of the oil-in-water emulsion as the immune adjuvant (promoting cytophagy)

The adjuvant vaccine composition was prepared by using macrophage strain RAW264.7 of mice as the antigen presenting cells and using OVA as antigens to be mixed with the oil-in-water emulsion. In vitro cell experiment was conducted to review the effect of the oil-in-water emulsion (prepared according to Example 11) on the antigen phagocytosis amount by the antigen presenting cells. The results show that the adjuvant group may significantly increase the antigen phagocytosis amount by RAW264.7 cells.

**Example 20** The oil-in-water emulsion as the drug release carrier

The oil-in-water emulsion prepared substantially according to the method of Example 4, with the exception of adding taxol having a mass concentration of 0.5 wt.% into the oil phase.

**Example 21** The oil-in-water emulsion as the drug release carrier

The oil-in-water emulsion prepared substantially according to the method of Example 5, with the exception of embedding insulin having a mass concentration of 1 wt.% in the PELA particles.

**Claims**

1. An oil-in-water emulsion containing no surfactant, **characterized in that** the emulsion comprises a metabolizable oil phase, an aqueous phase and oil-water amphipathic solid particles dispersed in the aqueous phase and having biocompatibility, wherein the oil phase comprises squalene or/and tocol; the aqueous phase is any one selected from the group consisting of purified water, water for injection, glycerine aqueous solution, buffering salts aqueous solution, or the combination of at least two selected therefrom; and the solid particles have an average particle size of from 50 nm to 10 $\mu$m, and are selected from the group consisting of poly(lactide-co-glycolide) particles, aluminium hydroxide particles, calcium phosphate particles, alginic acid particles coated with chitosan, calcium carbonate particles, polylactic acid particles, and chitosan particles, and the solid particles have a mass concentration of from 0.1 to 20 wt% in aqueous phase.

2. The oil-in-water emulsion claimed in claim 1, **characterized in that** the tocol is $\alpha$-tocopherol; preferably, the oil phase further comprises any vegetable oil, fish oil, animal oil or synthetic oil which is non-toxic to receptors and transformable by metabolism, preferably any one selected from the group consisting of soybean oil, miglitol, midchain oil, fish oil, vitamin E, vitamin E succinate, vitamin E acetate, safflower oil, corn oil, sea buckthron oil, linseed oil, peanut oil, tea-seed oil, sunflower seed oil, apricot kernel oil, coix seed oil, evening primrose seed oil, sesame oil, cottonseed oil, castor oil, low-erucic acid rapeseed oil, ethyl oleate, oleic acid, ethyl linoleate, isopropyl laurate, isopropyl myristate, ethyl butyrate, ethyl lactate, caprylic triglyceride or capric triglyceride, or the combination of at least two selected therefrom.

3. The oil-in-water emulsion claimed in claim 1 or 2, **characterized in that** the aqueous phase is any one selected from the group consisting of water for injection, phosphate buffering solution, citrate buffering solution or Tris buffer solution, or the combination of at least two selected therefrom;
preferably, the phosphate buffering solution, citrate buffering solution or Tris buffer solution independently has a pH value of from 5.0 to 8.1, preferably from 6.0 to 8.0.

4. The oil-in-water emulsion claimed in any of claims 1-3, **characterized in that** the aqueous phase comprises an univalent or polyvalent antigen, the antigen is any one selected from the group consisting of human antigens, non-human animal antigens, plant antigens, bacterial antigens, fungal antigens, viral antigens, parasite antigens and tumor antigens, or the combination of at least two selected therefrom;

preferably, the antigen is obtained from chick embryo culture, cell culture, body fluid, organ or tissue of a carrier by purification and separation, recombination gene expression or chemical synthesis, preferably the antigen is any one selected from the group consisting of attenuated vaccine, inactivated vaccine, split vaccine, subunit vaccine, polysaccharide conjugate vaccine, recombinant vaccine and DNA vaccine, or the combination of at least two selected therefrom;
preferably, the antigen is a viral antigen or antigenic preparation comprising at least three influenza seasonal strains, and optionally the viral antigen or antigenic preparation comprising at least one influenza virus strain related to pandemic outbreak or having the potential of being related to pandemic outbreak, wherein the influenza virus strain related to pandemic outbreak or having the potential of being related to pandemic outbreak is one selected from the group consisting of
human influenza viruses type A, B, C, including H1N1, H2N2, H3N2, H5N1, H7N7, H1N2, H9N2, H7N3, and H10N7;
swine influenza viruses H1N1, H1N2, H3N1 and H3N2;
dog or equine influenza viruses H7N7 and H3N8; or
avian influenza viruses H5N1, H7N2, H1N7, H7N3, H13N6, H5N9, H11N6, H3N8, H9N2, H5N2, H4N8, H10N7, H2N2, H8N4, H14N5, H6N5 and H12N5, or the combination of more than one selected therefrom;
preferably, the aqueous phase further comprises an officinal auxiliary substance, preferably pH modifier or/and buffering agent, further preferably one selected from the group consisting of sodium acetate, sodium lactate, sodium chloride, potassium chloride, calcium chloride, human serum protein, essential amino acid, non-essential amino acid, L-arginine monohydrochloride, sucrose, anhydrous D-mycose, mannitol, mannose, starch and gelatine, or the combination of at least selected therefrom.

5. The oil-in-water emulsion claimed in any of claims 1-4, **characterized in that**

the solid particles are the poly(lactide-co-glycolide) having a lactide-glycolide chain-segment molar ratio of from 10:90 to 90:10;
preferably, the solid particles are any one selected from the group consisting of aluminium hydroxide, calcium phosphate, calcium carbonate, chitosan, polylactic acid, or the mixture of at least two selected therefrom, further preferably any one selected from the group consisting of aluminium hydroxide, calcium phosphate, polylactic acid, or the mixture of at least two selected therfrom.

6. The oil-in-water emulsion claimed in any of claims 1-5, **characterized in that** the surfaces of solid particles are hydrophilically modified, hydrophobically modified, coated or graft-modified;

preferably, the surfaces or interiors of solid particles absorb, couple or embed the targeting substances, fluorescence indicators, isotope labelling substances, environmental response substances, cytokines, antibodies or immunomodulators;
preferably, the environmental response substances are selected from those with the pH-sensitive, heat sensitive or bioactivator sensitive groups;
preferably, the surfaces or interiors of solid particles absorb, couple or embed antigens;
preferably, the solid particles have an average particle size of from 1 nm to 10 $\mu$m, preferably from 10 nm to 5 $\mu$m;
preferably, the solid particles have a particle size distribution coefficient span value of less than 1.0;

preferably, the solid particles have a mass concentration of from 0.5 to 10 wt%, preferably from 1 to 8 wt%, in aqueous phase.

7. The oil-in-water emulsion claimed in any of claims 1-6, **characterized in that** the emulsion comprises a metabolizable oil phase, an aqueous phase and oil-water amphipathic solid particles dispersed in the aqueous phase and having biocompatibility, wherein the oil phase is squalene; the aqueous phase is any one selected from the group consisting of purified water, water for injection, glycerine aqueous solution, buffering salts aqueous solution or clinically usable transfusion, or the combination of at least two selected therefrom; and the solid particles are poly(lactide-co-glycolide) having an average particle size in a scale of from nanometer to micrometer;

preferably, the oil-in-water emulsion has an oil-water phase volume ratio of from 1:100 to 9:1, preferably from 1:50 to 1:2;
preferably, the emulsion droplets in the oil-in-water emulsion have an average particle size of from 50 nm to 300 $\mu$m, preferably from 100 nm to 100 $\mu$m;
preferably, the oil-in-water emulsion further comprises officinal additives comprising any one selected from the group consisting of diluents, stabilizers or preservatives, or the combination of at least two selected therefrom.

8. An immunogenic composition containing no surfactant, **characterized by** comprising (1) an antigen or antigen composition, and (2) an adjuvant composition, wherein said adjuvant composition comprises the oil-in-water emulsion claimed in any of claims 1-7.

**Patentansprüche**

1. Tensidfreie Öl-in-Wasser-Emulsion, **dadurch gekennzeichnet, dass** die Emulsion eine metabolisierbare Ölphase, eine wässrige Phase und amphipatische Öl-Wasser-Feststoffteilchen, die in der wässrigen Phase dispergiert sind und Biokompatibilität aufweisen, umfasst, wobei die Ölphase Squalen oder/und Tocol umfasst; die wässrige Phase ist irgendeine, die aus der Gruppe ausgewählt ist, die aus gereinigtem Wasser, Wasser zur Injektion, wässriger Glycerinlösung, wässriger Puffersalzlösung oder der Kombination aus mindestens zwei daraus ausgewählten besteht; und die Feststoffteilchen weisen eine mittlere Teilchengröße von 50 nm bis 10 $\mu$m auf und sind aus der Gruppe ausgewählt, die aus Poly(lactid-co-glycolid)teilchen, Aluminiumhydroxidteilchen, Calciumphosphatteilchen, Alginsäureteilchen, die mit Chitosan beschichtet sind, Calciumcarbonatteilchen, Polymilchsäureteilchen und Chitosanteilchen besteht, und die Feststoffteilchen weisen eine Massenkonzentration von 0, 1 bis 20 Gew.-% in wässriger Phase auf.

2. Öl-in-Wasser-Emulsion nach Anspruch 1, **dadurch gekennzeichnet, dass** das Tocol $\alpha$-Tocopherol ist; vorzugsweise umfasst die Ölphase ferner ein beliebiges pflanzliches Öl, Fischöl, tierisches Öl oder synthetisches Öl, das nicht toxisch für Rezeptoren ist und durch Stoffwechsel umgewandelt werden kann, vorzugsweise irgendeines, das aus der Gruppe ausgewählt ist, die aus Sojabohnenöl, Miglitol, Midchain-Öl, Fischöl, Vitamin E, Vitamin E-Succinat, Vitamin E-Acetat, Safloröl, Maisöl, Seebuckethronöl, Leinöl, Erdnussöl, Teesamenöl, Sonnenblumenkernöl, Aprikosenkernöl, Coixsamenöl, Nachtkerzensamenöl, Sesamöl, Baumwollsamenöl, Rizinusöl, erucasäurearmes Rapsöl, Ethyloleat, Ölsäure, Ethyllinoleat, Isopropyllaurat, Isopropylmyristat, Ethylbutyrat, Ethyllactat, Capryltriglycerid oder Caprintriglycerid oder der Kombination aus mindestens zwei daraus ausgewählten besteht.

3. Öl-in-Wasser-Emulsion nach Anspruch 1 oder 2, **dadurch gekennzeichnet, dass** die wässrige Phase irgendeine ist, die aus der Gruppe ausgewählt ist, die aus Wasser für Injektion, Phosphatpufferlösung, Citratpufferlösung oder Irispufferlösung oder der Kombination aus mindestens zwei daraus ausgewählten besteht; vorzugsweise weist die Phosphatpufferlösung, Citratpufferlösung oder Irispufferlösung unabhängig voneinander einen pH-Wert von 5,0 bis 8,1, vorzugsweise von 6,0 bis 8,0, auf.

4. Öl-in-Wasser-Emulsion nach einem der Ansprüche 1-3, **dadurch gekennzeichnet, dass** die wässrige Phase ein einwertiges oder mehrwertiges Antigen umfasst, wobei das Antigen irgendeines ist, das aus der Gruppe ausgewählt ist, die aus menschlichen Antigenen, nichtmenschlichen Tierantigenen, Pflanzenantigenen, bakteriellen Antigenen, Pilzantigenen, viralen Antigenen, Parasitenantigenen und Tumorantigenen oder der Kombination aus mindestens zwei daraus ausgewählten besteht; vorzugsweise wird das Antigen aus Hühnerembryokultur, Zellkultur, Körperflüssigkeit, Organ oder Gewebe eines Trägers durch Reinigung und Trennung, Rekombinationsgenexpression oder chemische Synthese erhalten, wobei das Antigen vorzugsweise irgendeines ist, das aus der Gruppe ausgewählt ist, die aus attenuiertem Impfstoff, inaktiviertem Impfstoff, Split-Impfstoff, Untereinheit-Impfstoff, Polysaccharidkonjugat-Impfstoff, rekombinantem Impfstoff und DNA-Impfstoff oder der Kombination aus mindestens zwei daraus ausgewählten besteht; vorzugsweise ist das Antigen ein virales Antigen oder eine antigene Zubereitung, umfassend mindestens drei saisonale Influenza-Stämme, und wobei das virale Antigen oder die antigene Zubereitung, gegebenenfalls mindestens einen Influenza-Virusstamm, der mit einem pandemischen Ausbruch in Zusammenhang steht oder das Potenzial aufweist, mit einem pandemischen Ausbruch in Zusammenhang zu stehen, umfasst, wobei der Influenza-Virusstamm, der mit einem pandemischen Ausbruch in Zusammenhang steht oder das Potenzial aufweist, mit einem pandemischen Ausbruch in Zusammenhang zu stehen, einer ist, der aus der Gruppe ausgewählt ist, die aus humanen Influenza-Viren vom Typ A, B, C, einschließlich H1N1, H2N2, H3N2, H5N1, H7N7, H1N2, H9N2, H7N3 und H10N7,

die Schweinegrippeviren H1N1, H1N2, H3N1 und H3N2, die Hunde- oder Pferdegrippeviren H7N7 und H3N8 oder die aviären Influenzaviren H5N1, H7N2, H1N7, H7N3, H13N6, H5N9, H11N6, H3N8, H9N2, H5N2, H4N8, H10N7, H2N2, H8N4, H14N5, H6N5 und H12N5 besteht oder die Kombinationen von mehr als einem daraus ausgewählten;

vorzugsweise umfasst die wässrige Phase ferner eine offizinale Hilfssubstanz, vorzugsweise einen pH-Modifikator oder/und ein Puffermittel, weiter vorzugsweise ausgewählt aus der Gruppe, die aus Natriumacetat, Natriumlactat, Natriumchlorid, Kaliumchlorid, Calciumchlorid, menschlichem Serumprotein, essentieller Aminosäure, nicht essentieller Aminosäure, L-Argininmonohydrochlorid, Saccharose, wasserfreier D-Mycose, Mannit, Mannose, Stärke und Gelatine oder der Kombination aus mindestens zwei daraus ausgewählten besteht.

5. Öl-in-Wasser-Emulsion nach einem der Ansprüche 1-4, **dadurch gekennzeichnet, dass** die Feststoffteilchen das Poly(lactid-co-glycolid) aufweisend ein Lactid-Glycolid-Kettensegment-Molverhältnis von 10:90 bis 90:10 sind; vorzugsweise sind die Feststoffteilchen irgendeines, das aus der Gruppe ausgewählt ist, die aus Aluminiumhydroxid, Calciumphosphat, Calciumcarbonat, Chitosan, Polymilchsäure oder der Mischung aus mindestens zwei daraus ausgewählten besteht, weiter vorzugsweise irgendeines, das aus der Gruppe ausgewählt ist, die aus Aluminiumhydroxid, Calciumphosphat, Polymilchsäure oder der Mischung aus mindestens zwei daraus ausgewählten besteht.

6. Öl-in-Wasser-Emulsion nach einem der Ansprüche 1-5, **dadurch gekennzeichnet, dass** die Oberflächen von Feststoffteilchen hydrophil modifiziert, hydrophob modifiziert, beschichtet oder pfropfmodifiziert sind;

vorzugsweise absorbieren, koppeln oder betten die Oberflächen oder Innenräume von Feststoffteilchen die Zielsubstanzen, Fluoreszenzindikatoren, Isotopenmarkierungssubstanzen, Umweltreaktionssubstanzen, Zytokine, Antikörper oder Immunmodulatoren ein;
vorzugsweise sind die Umweltreaktionssubstanzen aus solchen mit den pH-empfindlichen, wärmeempfindlichen oder Bioaktivator-empfindlichen Gruppen ausgewählt; vorzugsweise absorbieren, koppeln oder betten die Oberflächen oder Innenräume von Feststoffteilchen Antigene ein;
vorzugsweise weisen die Feststoffteilchen eine mittlere Teilchengröße von 1 nm bis 10 pm, vorzugsweise von 10 nm bis 5 μm auf;
vorzugsweise weisen die Feststoffteilchen einen Kalibrierwert des Teilchengrößenverteilungskoeffizienten von weniger als 1,0 auf;
vorzugsweise weisen die Feststoffteilchen eine Massenkonzentration von 0,5 bis 10 Gew.-%, vorzugsweise von 1 bis 8 Gew.-%, in wässriger Phase auf.

7. Öl-in-Wasser-Emulsion nach einem der Ansprüche 1-6, **dadurch gekennzeichnet, dass** die Emulsion eine metabolisierbare Ölphase, eine wässrige Phase und amphipatische Öl-Wasser-Feststoffteilchen umfasst, die in der wässrigen Phase dispergiert sind und Biokompatibilität aufweisen, wobei die Ölphase Squalen ist; die wässrige Phase ist irgendeine, die aus der Gruppe ausgewählt ist, die aus gereinigtem Wasser, Wasser für Injektion, wässriger Glycerinlösung, wässriger Puffersalzlösung oder klinisch verwendbarer Transfusion oder der Kombination aus mindestens zwei daraus ausgewählten besteht; und die Feststoffteilchen sind Poly(lactid-co-glycolid) aufweisend eine mittlere Teilchengröße in einer Skala von Nanometer bis Mikrometer; vorzugsweise weist die Öl-in-Wasser-Emulsion ein Öl-Wasser-Phasenvolumenverhältnis von 1:100 bis 9: 1, vorzugsweise von 1:50 bis 1:2, auf;

vorzugsweise weisen die Emulsionströpfchen in der Öl-in-Wasser-Emulsion eine mittlere Teilchengröße von 50 nm bis 300 pm, vorzugsweise von 100 nm bis 100 pm, auf;
vorzugsweise umfasst die Öl-in-Wasser-Emulsion ferner offizinale Additive, die irgendeines umfassen, das aus der Gruppe ausgewählt ist, die aus Verdünnungsmitteln, Stabilisatoren oder Konservierungsmitteln oder der Kombination aus mindestens zwei daraus ausgewählten besteht.

8. Tensidfreie immunogene Zusammensetzung, **dadurch gekennzeichnet, dass** sie (1) ein Antigen oder eine Antigenzusammensetzung und (2) eine Adjuvanszusammensetzung umfasst, wobei die Adjuvanszusammensetzung die Öl-in-Wasser-Emulsion nach einem der Ansprüche 1-7 umfasst.

## Revendications

1. Émulsion huile dans eau ne contenant pas de tensioactif, **caractérisée en ce que** l'émulsion comprend une phase huileuse métabolisable, une phase aqueuse et des particules solides amphipathiques huile-eau dispersées dans la phase aqueuse et présentant une biocompatibilité, dans laquelle la phase huileuse comprend du squalène ou/et du tocol ; la phase aqueuse est l'un quelconque des éléments choisi dans le groupe constitué par l'eau purifiée, l'eau pour injection, la solution aqueuse de glycérine, la solution aqueuse de sels tampons, ou la combinaison d'au moins deux éléments choisis parmi ceux-ci ; les particules solides ont une taille moyenne de particule comprise entre 50 nm et 10 μm et sont choisies dans le groupe constitué par les particules de poly(lactide-co-glycolide), les particules d'hydroxyde d'aluminium, les particules de phosphate de calcium, les particules d'acide alginique enrobées de chitosane, les particules de carbonate de calcium, les particules d'acide polylactique et les particules de chitosane, et les particules solides ont une concentration massique comprise entre 0, 1 et 20 % en poids dans la phase aqueuse.

2. Émulsion huile dans eau selon la revendication 1, **caractérisée en ce que** le tocol est l'α-tocophérol ; de préférence, la phase huileuse comprend de plus une huile végétale, une huile de poisson, une huile animale ou une huile synthétique étant non toxique pour les récepteurs et transformable par le métabolisme, de préférence un élément quelconque choisi dans le groupe constitué par l'huile de soja, le miglitol, l'huile à chaîne moyenne, l'huile de poisson, la vitamine E, le succinate de vitamine E, l'acétate de vitamine E, l'huile de carthame, l'huile de maïs, l'huile d'argousier, l'huile de lin, l'huile d'arachide, l'huile de graines de thé, l'huile de graines de tournesol, l'huile de noyaux d'abricot, l'huile de graines de larmes de Job, l'huile de graines d'onagre, l'huile de sésame, l'huile de graines de coton, l'huile de ricin, l'huile de colza à faible teneur en acide érucique, l'oléate d'éthyle, l'acide oléique, le linoléate d'éthyle, le laurate d'isopropyle, le myristate d'isopropyle, le butyrate d'éthyle, le lactate d'éthyle, le triglycéride caprylique ou le triglycéride caprique, ou la combinaison d'au moins deux éléments choisis parmi ceux-ci.

3. Émulsion huile dans eau selon la revendication 1 ou 2, **caractérisée en ce que** la phase aqueuse est une quelconque solution choisie dans le groupe constitué par l'eau pour injection, la solution tampon de phosphate, la solution tampon de citrate ou la solution tampon d'iris, ou la combinaison d'au moins deux solutions choisies parmi celles-ci ; de préférence, la solution tampon de phosphate, la solution tampon de citrate ou la solution tampon d'iris ont indépendamment une valeur de pH comprise entre 5,0 et 8,1, de préférence entre 6,0 et 8,0.

4. Émulsion huile dans eau selon l'une quelconque des revendications 1-3, **caractérisée en ce que** la phase aqueuse comprend un antigène univalent ou polyvalent, l'antigène étant un antigène quelconque choisi dans le groupe constitué par des antigènes humains, des antigènes d'animaux non humains, des antigènes végétaux, des antigènes bactériens, des antigènes fongiques, des antigènes viraux, des antigènes parasitaires et des antigènes tumoraux, ou la combinaison d'au moins deux antigènes choisis parmi ceux-ci ;

de préférence, l'antigène est obtenu à partir d'une culture d'embryon de poulet, d'une culture cellulaire, d'un fluide corporel, d'un organe ou d'un tissu d'un vecteur par purification et séparation, expression génique par recombinaison ou synthèse chimique, de préférence l'antigène est l'un quelconque des vaccins sélectionnés dans le groupe constitué par les vaccins atténués, les vaccins inactivés, les vaccins fractionnés, les vaccins à sous-unités, les vaccins conjugués polysaccharidiques, les vaccins recombinants et les vaccins à ADN, ou la combinaison d'au moins deux vaccins choisis parmi ceux-ci ;
de préférence, l'antigène est un antigène viral ou une préparation antigénique comprenant au moins trois souches saisonnières de grippe, et éventuellement l'antigène viral ou la préparation antigénique comprenant au moins une souche de virus grippal liée à un foyer pandémique ou ayant le potentiel d'être liée à un foyer pandémique, dans laquelle la souche de virus grippal liée à un foyer pandémique ou susceptible d'être liée à un foyer pandémique est choisie dans le groupe constitué des virus grippaux humains de type A, B, C, y compris H1N1, H2N2, H3N2, H5N1, H7N7, H1N2, H9N2, H7N3, et H10N7 ;
les virus de la grippe porcine H1N1, H1N2, H3N1 et H3N2 ;
les virus de la grippe canine ou équine H7N7 et H3N8 ; ou
les virus de la grippe aviaire H5N1, H7N2, H1N7, H7N3, H13N6, H5N9, H11N6, H3N8, H9N2, H5N2, H4N8, H10N7, H2N2, H8N4, H14N5, H6N5 et H12N5, ou la combinaison de plus d'un de ces virus choisis parmi ceux-ci ;
de préférence, la phase aqueuse comprend de plus une substance auxiliaire officinale, de préférence un modificateur de pH et/ou un agent tampon, de préférence encore un élément choisi dans le groupe constitué par l'acétate de sodium, le lactate de sodium, le chlorure de sodium, le chlorure de potassium, le chlorure de calcium, la protéine sérique humaine, l'acide aminé essentiel, l'acide aminé non essentiel, le monohydrochlorure de L-arginine, le saccharose, le D-mycose anhydre, le mannitol, le mannose, l'amidon et la gélatine, ou la combinaison d'au moins deux éléments choisis parmi ceux-ci.

**5.** Émulsion huile dans eau selon l'une quelconque des revendications 1-4, **caractérisée en ce que** les particules solides sont des poly(lactide-co-glycolide) dont le rapport molaire entre les segments de chaîne lactide-glycolide est compris entre 10:90 et 90:10 ;

de préférence, les particules solides sont une quelconque particule choisie dans le groupe constitué par l'hydroxyde d'aluminium, le phosphate de calcium, le carbonate de calcium, le chitosane, l'acide polylactique ou le mélange d'au moins deux éléments choisis parmi ceux-ci, de préférence encore une quelconque particule choisie dans le groupe constitué par l'hydroxyde d'aluminium, le phosphate de calcium, l'acide polylactique ou le mélange d'au moins deux éléments choisi parmi ceux-ci.

**6.** Émulsion huile dans eau selon l'une quelconque des revendications 1-5, **caractérisée en ce que** les surfaces des particules solides sont modifiées de manière hydrophile, de manière hydrophobe, enduites ou modifiées par greffage ;

de préférence, les surfaces ou les intérieurs des particules solides absorbent, couplent ou intègrent les substances de ciblage, les indicateurs de fluorescence, les substances de marquage isotopique, les substances de réponse environnementale, les cytokines, les anticorps ou les immunomodulateurs ; de préférence, les substances de réponse environnementale sont choisies parmi celles avec les groupes qui sont sensibles au pH, à la chaleur ou aux bioactivateurs ;

de préférence, les surfaces ou les intérieurs des particules solides absorbent, couplent ou intègrent les antigènes ;

de préférence, les particules solides ont une taille moyenne de particule comprise entre 1 nm et 10 pm, de préférence entre 10 nm et 5 $\mu$m ;

de préférence, les particules solides ont un coefficient de distribution de la taille des particules d'une valeur d'échelle inférieure à 1,0 ;

de préférence, les particules solides ont une concentration massique de 0,5 à 10 % en poids, de préférence de 1 à 8 % en poids, en phase aqueuse.

**7.** Émulsion huile dans eau selon l'une quelconque des revendications 1-6, **caractérisée en ce que** l'émulsion comprend une phase huileuse métabolisable, une phase aqueuse et des particules solides amphipathiques huile-eau dispersées dans la phase aqueuse et présentant une biocompatibilité, dans laquelle la phase huileuse est du squalène ; la phase aqueuse est l'un quelconque des éléments choisi dans le groupe constitué par l'eau purifiée, l'eau pour injection, la solution aqueuse de glycérine, la solution aqueuse de sels tampons ou la transfusion cliniquement utilisable, ou la combinaison d'au moins deux éléments choisis parmi ceux-ci ; et les particules solides sont des poly(lactide-co-glycolide) ayant une taille moyenne de particule sur une échelle allant du nanomètre au micromètre ;

de préférence, l'émulsion huile dans eau a un rapport volumétrique entre la phase huileuse et la phase aqueuse compris entre 1:100 et 9:1, de préférence entre 1:50 et 1:2 ;

de préférence, les gouttelettes de l'émulsion huile dans eau ont une taille moyenne de particule de 50 nm à 300 pm, de préférence de 100 nm à 100 $\mu$m ;

de préférence, l'émulsion huile dans eau comprend de plus des additifs officinaux comprenant des éléments quelconque choisis dans le groupe constitué par les diluants, les stabilisateurs ou les conservateurs, ou la combinaison d'au moins deux additifs choisis parmi ceux-ci.

**8.** Composition immunogène ne contenant pas de tensioactif, **caractérisée en ce qu'**elle comprend (1) un antigène ou une composition d'antigènes, et (2) une composition d'adjuvants, dans laquelle ladite composition d'adjuvants comprend l'émulsion huile dans eau selon l'une quelconque des revendications 1-7.

Fig.1

Volume-Particle Size Distribution

Fig.2

Fig.3

Fig.4

Fig.5

Volume-Particle Size Distribution

Fig.6

Fig.7

Fig.8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 101267835 A
- CN 102293743 B
- CN 101365485 A

### Non-patent literature cited in the description

- **ZHUO AO ; ZHI YANG ; JIANFANG WANG ; GUANGZHAO ZHANG ; TO NGAI.** Emulsion-Templated Liquid Core-Polymer Shell Microcapsule Formation. *Langmuir,* 2009, vol. 25, 2572-2574
- **CATHERINE P. WHITBY ; LI HUI LIM ; NASRIN GHOUCHI ESKANDAR ; SPOMENKA SIMOVIC ; CLIVE A. PRESTIDGE.** Poly(lactic-co-glycolic acid) as a particulate emulsifier. *Journal of Colloid and Interface Science,* 2012, vol. 375, 142-147
- **ZENGJIANG WEI ; CHAOYANG WANG ; HAO LIU ; SHENGWEN ZOU ; ZHEN TONG.** Facile fabrication of biocompatible PLGA drug-carrying microspheres by O/W pickering emulsions. *Colloids and Surfaces B: Biointerfaces,* 2012, vol. 91, 97-105
- *CHEMICAL ABSTRACTS,* 111-02-4
- **DORLAND ; W. B. SANDERS.** *Medical Dictionary,* 1974